# EUROPÄISCHE PATENTSCHRIFT

(11) **EP 2 547 200 B1**
(45) Veröffentlichungstag und Bekanntmachung des Hinweises auf die Patenterteilung: **25.02.2015**
(21) Anmeldenummer: 11708047.3
(22) Anmeldetag: 14.03.2011
(51) Int. Cl.: A01N 25/00

(54) **ZUSAMMENSETZUNG UMFASSEND EIN PESTIZID UND EIN ALKOXYLAT VON VERZWEIGTEM NONYLAMIN**
COMPOSITION COMPRISING A PESTICIDE AND AN ALKOXYLATE OF BRANCHED NONYLAMINE
COMPOSITION COMPRENANT UN PESTICIDE ET UN ALCOXYLATE DE NONYLAMINE RAMIFIÉ

(30) Priorität: 17.03.2010 EP 10156710
(43) Veröffentlichungstag der Anmeldung: 23.01.2013
(73) Patentinhaber: BASF SE, 67056 Ludwigshafen (DE)
(72) Erfinder: KLINGELHOEFER, Paul, 68165 Mannheim (DE); MAITRO-VOGEL, Sophie, 68199 Mannheim (DE); HUYGHE, Kevin, 2950 Kapellen (BE); HADERLEIN, Gerd, 67269 Grünstadt (DE); SCHNABEL, Gerhard, 63820 Elsenfeld (DE); NOLTE, Marc, 68165 Mannheim (DE); EVANS, Richard, Roger, 67117 Limburgerhof (DE)
(86) Internationale Anmeldenummer: PCT/EP2011/053781
(87) Internationale Veröffentlichungsnummer: WO 2011/113786

(56) Entgegenhaltungen:
- EP-B1- 1 326 493
- WO-A2-2011/104211
- DE-A1- 4 037 735
- US-A- 2 689 263
- US-A- 5 118 444
- US-A- 5 317 003
- US-A- 5 710 104
- US-A1- 2007 049 498
- US-A1- 2010 317 521
- WYRILL J B ET AL: "GLYPHOSATE TOXICITY TO COMMON MILKWEED AND HEMP DOGBANE AS INFLUENCED BY SURFACTANTS", WEED SCIENCE, WEED SCIENCE SOCIETY OF AMERICA, CHAMPAIGN, IL, US, Bd. 25, Nr. 3, 1. Mai 1977 (1977-05-01), Seiten 275-287, XP002034447, ISSN: 0043-1745
- PETER L. DEBENNEVILLE ET AL: "Research ArticleThe Behavior of Aliphatic Aldehydes in the Leuckart-Wallach Reaction", JOURNAL OF THE AMERICAN CHEMICAL SOCIETY, Bd. 72, Nr. 7, 19. Juli 1950 (1950-07-19), Seiten 3073-3075, XP55010472, ISSN: 0002-7863, DOI: 10.1021/ja01163a074
- H. Walther ET AL: "Aldehyde, tert. Amine bzw. Acetale aus Amidacetalen und Grignardverbindungen", , 3. Februar 1980 (1980-02-03), Seiten 902-908, XP55010475, Germany DOI: 10.1002/prac.19803220607 Gefunden im Internet: URL:http://onlinelibrary.wiley.com/doi/10. 1002/prac.19803220607/pdf [gefunden am 2011-10-26]
- L. A. WALTER ET AL: "The Reduction of Cyanides", JOURNAL OF THE AMERICAN CHEMICAL SOCIETY, Bd. 56, Nr. 7, 1. Juli 1934 (1934-07-01), Seiten 1614-1616, XP55010478, ISSN: 0002-7863, DOI: 10.1021/ja01322a051
- PATRICK&EMSP14;J.&EMSP14;M. STALS ET AL: "Asymmetrically Substituted Benzene-1,3,5-tricarboxamides: Self-Assembly and Odd-Even Effects in the Solid State and in Dilute Solution", CHEMISTRY - A EUROPEAN JOURNAL, Bd. 15, Nr. 9, 16. Februar 2009 (2009-02-16), Seiten 2071-2080, XP55010481, ISSN: 0947-6539, DOI: 10.1002/chem.200802196

## Beschreibung

Gegenstand der vorliegenden Erfindung ist eine Zusammensetzung umfassend ein Pestizid und ein Alkoxylat. Außerdem betrifft die Erfindung das Alkoxylat, ein Verfahren zu seiner Herstellung, und seine Verwendung als Hilfsmittel in Pestizid-haltigen Spritzbrühen. Die Erfindung betrifft weiterhin ein Verfahren zur Bekämpfung von phytopathogenen Pilzen und/oder unerwünschtem Pflanzenwuchs und/oder unerwünschtem Insekten- oder Milbenbefall und/oder zur Regulation des Wachstums von Pflanzen, wobei man die Zusammensetzung auf die jeweiligen Schädlinge, deren Lebensraum oder die vor dem jeweiligen Schädüng zu schützenden Pflanzen, den Boden und/oder auf unerwünschte Pflanzen und/oder die Nutzpflanzen und/oder deren Lebensraum einwirken lässt. Ferner betrifft die Erfindung Saatgut enthaltend die Zusammensetzung. Kombinationen bevorzugter Merkmale mit anderen bevorzugten Merkmalen werden von der vorliegenden Erfindung umfasst.

Alkoxylate und ihre Verwendung in agrochemischen Formulierungen als Adjuvantien sind allgemein bekannt:
WO 2009/004044 offenbart eine herbizide Zusammensetzung umfassend ein Phenoxysäure Herbizid und ein alkoxyliertes Alkylamin als Adjuvants.
US 5,668,085 offenbart eine herbizide Zusammensetzung umfassend eine wässrige Lösung von Glyphosat und Tensid. Das Tensid kann ein alkoxyliertes Alkylamin sein, wobei die Alkylgruppe 8 bis 22 Kohlenstoffatome enthält.
WO 2001/97614 offenbart herbizide Mittel enthaltend ein Herbizid und ein Tensid, welches beispielsweise ein alkoxiliertes Alkylamin sein kann.
DE 10 2005 037 971 offenbart Zusammensetzungen enthaltend Fettalkoholalkoxlyate und tertiäre Amine, die Alkoxygruppen enthalten. Diese Mischung kann als Wirkverbesserer in agrochemischen Formulierungen eingesetzt werden.
US2007049498 beschreibt agrochemische Zusammensetzungen, die ein Aminalkoxylat mit einem C₈₋₂₂-Alkyl- oder C₈₋₂₂-Alkenylrest enthalten.
US5118444 beschreibt agrochemische Zusammensetzungen, die ein Aminoxidalkoxylat mit einem C₆₋₂₀-Alkyl- oder C₆₋₂₀-Alkenylrest enthalten.
US5317003 beschreibt herbizide Zusammensetzungen, die ein Aminalkoxylat mit einem C₆₋₁₄-Alkyl- oder C₆₋₁₄-Alkenylrest enthalten.
EP1326493 beschreibt agrochemische Zusammensetzungen, die ein Aminoxidalkoxylat mit einem C₈-Alkylest enthalten.

In Wyrill J B et al.: WEED SCIENCE, WEED SCIENCE SOCIETY OF AMERICA, CHAMPAIGN, IL, US, Bd. 25, Nr. 3, 1. Mai 1977 (1977-05-01), Seiten 275-287 sind eine Reihe von Aminethoxylaten und ihr Einfluss auf die Toxizität von Glyphosate beschrieben.

US5710104 betrifft Glyphosate-Formulierungen die bestimmte Alkoholalkoxylate enthalten.

In US2689263 ist die Herstellung von Hydroxyalkylaminen beschrieben.

Debenneville et al.: JOURNAL OF THE AMERICAN CHEMICAL SOCIETY, Bd. 72, Nr. 7, 19. Juli 1950, Seiten 3073-3075 befasst sich mit der Leuckart-Wallach-Reaktion, bei der 3,5,5-Trimethylhexaldehyd mit Methylnonylamin umgesetzt wird.

H. Walther et al.: Journal f. prakt. Chemie, Band 322, Heft 6, 1980, Seiten 902-908, beschreibt die Herstellung von Aldehyden, tertiären Aminen und Acetalen aus Aminacetalen und Grignardverbindungen.
In der DE 4037735 sind Amine wie 2,2-Dimethylheptylamin, 2-Methyl-2-(n-propyl)-pentylamin und 2-Ethyl-2-(n-propyl)-pentylamin beschrieben.

L. A. Walter et al.: JOURNAL OF THE AMERICAN CHEMICAL SOCIETY, Bd. 56, Nr. 7, 1. 1934, Seiten 1614-1616 befasst sich mit der Reduktion von Cyaniden zu Aminen.

In Stals et al.: CHEMISTRY - A EUROPEAN JOURNAL, Bd. 15, Nr. 9, Februar 2009, Seiten 2071-2080, ist die Synthese von enantiomerenreinem 2-Methyloctylamin beschrieben.

WO 2011/104211 beschreibt Tensidgemische zum Einsatz in Herbizidformulierungen. Unter den Tensiden sind quaternisierte Amine mit einem C₆-C₂₂- und einem C₁-C₄-Alkylrest genannt. Es wird eine Vielzahl von Alkylresten aufgezählt, darunter auch Isononyl.

Alkoxylierte Alkylamine, insbesondere kommerziell erhältliche ethoxylierte Taigfettamine (POEA), haben kritische toxische (wie Haut- und Augenreizung) und ökotoxische Eigenschaften (wie hohe Ecotoxizität gegen Wasserorganismen wie Algen und Daphnien). So gilt beispielsweise das in Roundup® Herbiziden oft als Netzmittel enthaltene POEA (CAS Nr. 61791-26-2) als verhältnismäßig aquatoxisch (Tsui und Chu, Chemosphere 2003, 52, 1189-1197).

Aufgabe der vorliegende Erfindung war daher ein Adjuvants zu finden, das für Herbizide wie Glyphosat gut geeignet ist und dabei weniger toxisch (v.a. weniger aquatoxisch) ist. Weiterhin sollte das Adjuvants eine lagerstabile Formulierung der Pestizide ermöglichen.

Die Aufgabe wurde gelöst durch eine Zusammensetzung umfassend ein Pestizid mit mindestens einer H-aciden Gruppe oder dessen anionisches Salz und ein Alkoxylat, dadurch gekennzeichnet, dass das Alkoxlyat ein Aminalkoxylat (A) 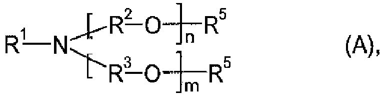 oder ein quaternisiertes Derivat (AQ) 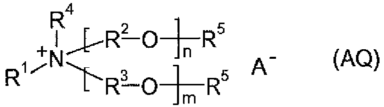 des Aminalkoxylats (A) ist, wobei
- R¹: ein verzweigter, aliphatischer Alkylrest C₉H₁₉ ist,
- R², R³, und R⁷: unabhängig voneinander Ethylen, Propylen, Butylen oder eine Mischung davon sind,
- R⁴: ein H, -OH, -OR⁶, -[R⁷-O]ₚ-R⁵ oder ein Sauerstoffanion ist,
- R⁵: ein H, C₁-C₆-Alkyl, C₂-C₆-Alkenyl, C₂-C₆-Alkinyl, -SO₃R^{a}, -P(O)OR^{b}OR^{c}, -CH₂CO₂R^{d}, oder -C(O)R^{e} ist,
- R⁶: ein C₁-C₆-Alkyl, C₂-C₆-Alkenyl, oder C₂-C₆Alkinyl ist,
- R^{a} und R^{d}: unabhängig voneinander ein H, anorganische oder organische Kationen sind,
- R^{b} und R^{c}: unabhängig voneinander ein H, anorganische oder organische Kationen, C₁-C₆-Alkyl, C₂-C₆-Alkenyl oder C₂-C₆-Alkinyl sind,
- R^{e}: C₁-C₂₂-Alkyl, C₂-C₂₂-Alkenyl, C₂-C₂₂-Alkinyl, C₆-C₂₂-Aryl, C₇-C₂₂-Alkylaryl ist,
- n, m und p: unabhängig voneinander ein Wert von 1 bis 30 haben, und
- A⁻: ein landwirtschaftlich akzeptables Anion oder, falls R³ ein Sauerstoffanion ist, A⁻nicht vorhanden ist.

Bevorzugt umfasst die erfindungsgemäße Zusammensetzung ein Pestizid und ein Alkoxylat, wobei das Alkoxylat ein Aminalkoxylat (A) ist.

Der Rest R¹ ist ein verzweigter, aliphatischer Alkylrest C₉H₁₉. Beispiele für R¹ sind unter anderem 2-Methyl-1-octyl, 2-Ethyl-1-heptanyl, 2-Propyl-1-hexanyl, 3-Methyl-2-propyl-pentyl, 2-Methyl-2-ethyl-hexal, oder 2,3-Dimethyl-heptyl. Bevorzugt umfasst R¹ mehrere verschiedene, verzweigte, aliphatische Alkylreste C₉H₁₉. Der mittlerer Verzweigungsgrad von R¹ beträgt meist 1,01 bis 2,5, bevorzugt 1,05 bis 1,8, besonders bevorzugt 1,1 bis 1,5, ganz besonders bevorzugt 1,2 bis 1,3.

Der Begriff "Verzweigungsgrad" wird hierbei in prinzipiell bekannter Art und Weise als die Zahl der Methylgruppen in einem Molekül des Alkohols abzüglich 1 definiert. Der mittlere Verzweigungsgrad ist der statistische Mittelwert der Verzweigungsgrade aller Moleküle einer Probe. Mit anderen Worten gesagt, kann es sich bei dem eingesetzten Rest R¹ um ein Gemisch verschiedener Alkylgruppen C₉H₁₉ handeln. Dementsprechend kann es sich bei dem als Ausgangsmaterial für die Synthese eingesetzten Alkohols R¹-OH um ein Gemisch verschiedener Alkohole handeln.

Der mittlere Verwzweigungsgrad kann wie folgt ¹H-NMR-spektroskopisch ermittelt werden: Dazu wird eine Probe des Alkohols R¹-OH zunächst einer Derivatisierung mit Trichloracetylisocyanat (TAI) unterzogen. Dabei werden die Alohole in die Carbaminsäureester überführt. Die Signale der veresterten primären Alkohole liegen bei δ□ = 4,7 bis 4,0 ppm, die der (soweit vorhanden) veresterten sekundären Alkohole bei etwa 5 ppm und in der Probe vorhandenes Wasser reagiert mit TAI zur Carbaminsäure ab. Alle Methyl-, Methylen- und Methinprotonen liegen im Bereich von 2,4 bis 0,4 ppm. Die Signale < 1 ppm sind dabei den Methylgruppen zugeordnet. Aus dem so erhalten Spektrum läßt sich der mittlere Verzweigungsgrad (Iso-Index) wie folgt berechnen:

Iso-Index = ((F(CH₃) / 3) / (F(CH2-OH) / 2)) - 1

wobei F(CH₃) für die den Methylprotonen entsprechende Signalfläche und F(CH₂-OH) für die Signalfiäche der Methylenprotonen in der CH₂-OH-Gruppe steht.

Bevorzugt hat n ein Wert von 1 bis 20, besonders bevorzugt von 1 bis 15. Bevorzugt hat m ein Wert von 1 bis 20, besonders bevorzugt von 1 bis 15. Bevorzugt hat p ein Wert von 1 bis 30, besonders bevorzugt von 1 bis 20. Die Werte von n, m und o sind normalerweise durchnittliche Werte, wie sie meist bei der Alkoxylierung mit Alkoxiden entstehen. Daher können n, m und o sowohl ganzzahlige Werte als auch alle Werte zwischen den ganzen Zahlen einnehmen.

Bevorzugt ist beim Aminalkoxlat (A) ist die Summe aus n und m 2 bis 40, und bei dessen quarternisiertes Derivat (AQ) ist die Summe aus n, m und p 3 bis 80.

Beim Aminalkoxlat (A) ist die Summe aus n und m ist besonders bevorzugt 3 bis 30, und speziell 5 bis 25. In einer weiteren speziell bevorzugten Ausführungsform ist die Summe aus n und m 6 bis 9, insbesondere 6,5 bis 8,5 und insbesondere 6,9 bis 7,9. In einer weiteren speziell bevorzugten Ausführungsform ist die Summe aus n und m 11 bis 40, insbesondere 12 bis 30 und insbesondere 13,5 bis 25. In einer weiteren speziell bevorzugten Ausführungsform ist die Summe aus n und m 8 bis 13, insbesondere 9 bis 11.

Beim quarternisierten Derivat (AQ) des Aminalkoxlat (A) ist die Summe aus n, m und p besonders bevorzugt 3 bis 40, und speziell 5 bis 25. In einer speziell bevorzugten Ausführungsform ist die Summe aus n, m und p 8 bis 13, insbesondere 9 bis 11.

R², R³ und R⁷ sind bevorzugt unabhängig voneinander Ethylen, Ethylen und Propylen, Ethylen und Butylen, oder Ethylen, Propylen und Butylen. In einer weiteren bevorzugten Ausführungsform sind R², R³ und R⁷ Propylen. In einer weiteren bevorzugten Ausführungsform sind R², R³ und R⁷ Butylen. Besonders bevorzugt sind R², R³ und R⁷ unabhängig voneinander Ethylen, oder Ethylen und Propylen. Ganz besonders bevorzugt sind R², R³ und R⁷ Ethylen.

Wenn R², R³ oder R⁷ einen Butylenrest enthalten, kann dieser als n-Butylen, iso-Butylen, oder 2,3-Butylengruppe vorliegen, wobei n-Butylen und iso-Butylen bevorzugt sind und n-Butylen am meisten bevorzugt ist.

R², R³, und R⁷ können unabhängig voneinander eine Mischung von Ethylen, Propylen, oder Butylen sein. Dabei kann zum Beispiel einer oder können alle Reste R², R³, und R⁷ jeweils in einer Alkoxlyatkette eine Mischung dieser Gruppen enthalten. Solche Mischungen können in beliebiger Reihenfolge miteinander verbunden sein, beispielsweise statistisch oder in Blockweise (wie ein Block Ethylen und ein Block Propylen). Es können auch jeweils einer oder mehrere der Reste R², R³, und R⁷ eine komplette Alkoxlyatkette aus verschiedenen Alkylengruppen aufgebaut sein. Beispielsweise kann R² und R³ aus Ethylen und R⁷ aus Propylen aufgebaut sein.

R⁴ ist bevorzugt ein H, OHoder ein Sauerstoffanion, besonders bevorzugt ist es ein Hoder ein Sauerstoffanion. In einer speziell bevorzugten Ausführungsform ist R⁴ ein Sauerstoffanion. In einer weiteren speziell bevorzugten Ausführungsform ist R⁴ ein H.

R⁵ ist bevorzugt ein H oder C₁-C₆ Alkyl, besonders bevorzugt ein H oder Methyl, insbesondere H.

R⁶ ist bevorzugt ein C₁-C₆ Alkyl, wie Methyl.

R^{a} und R^{d} sind unabhängig voneinander H, oder anorganische oder organische Kationen, welche einfach oder mehrfach positiv geladen sein können. Beispiele für anorganische Kationen sind Kationen von Ammonium, Na⁺, K⁺, Mg²⁺, Ca²⁺, oder Zn²⁺. Beispiele für organische Kationen sind Methylammonium, Dimethylammonium, Trimethylammonium, Tetramethylammonium, (2-Hydroxyethyl)ammonium, Bis(2-hydroxyethyl)-ammonium, Tris(2-hydroxyethyl)ammonium, Tetra(2-hydroxyethyl)ammonium. Bevorzugt sind R^{a} und R^{d} sind unabhängig voneinander H, oder anorganische Kationen. Wenn ein anorganisches oder organisches Kation vorliegt, dann würde die zugehörige anionische Gruppe von der entsprechenden funktionellen Guppe (z.B. -SO₃-, -P(O)O-O-, oder -CH₂CO₂⁻) an R⁶ gebildet werden.

R^{b} und R^{c} sind bevorzugt unabhängig voneinander H, anorganische oder organische Kationen. Geeignete anorganische oder organische Kationen sind die unter R^{a} genannten.

In einer weiteren Ausführungsform können beim quarternären Derivat (AQ) die Reste R^{a}, R^{b}, R^{c} und R^{d} unabhängig voneinander organische Kationen sein, wobei die kationische Gruppe das quarternäre Stickstoff-Kation von AQ selber ist. Dabei könnte AQ also ein Zwitterion bilden, wobei die anionische Gruppe von der entsprechenden funktionellen Gruppe (z.B. -SO₃⁻, -P(O)O-O-, oder -CH₂CO₂⁻) an R⁶ in AQ gebildet wird, und die kationische Gruppe von dem quarternären Stickstoff von AQ. In dieser Zwitterionenform von AQ ist die Anwesenheit eines landwirtschaftlich akzeptablen Anions A⁻optional.

R^{e} ist bevorzugt C₁-C₁₂-Alkyl, C₁-C₁₂-Aryl, oder C₁-C₁₂-Alkylaryl, besonders bevorzugt C₁-C₆-Alkyl.

A⁻ ist ein landwirtschaftlich akzeptables Anion, wie sie dem Fachmann allgemein bekannt sind. Bevorzugt ist A- ein Halogenid (wie Chlorid oder Bromid), Phosphat, Sulfat oder ein anionisches Pestizid. Auch Propionat, Acetat oder Formiat sind als A- geeignet. Besonders bevorzugt ist A⁻ ein anionisches Pestizid, wie Glyphosatanion oder Glufosinatanion. Falls R⁴ ein Sauerstoffanion ist, liegt ein Aminoxid vor. In diesem Fall ist ein weiteres Anion wie A⁻ nicht vorhanden.

Beim Aminalkoxylat (A) sind bevorzugt R² und R³ unabhängig voneinander Ethylen, Ethylen und Propylen, Ethylen und Butylen, oder Ethylen, Propylen und Butylen, und die Summe aus n und m ist 2 bis 60, bevorzugt 2 bis 40, besonders bevorzugt 3 bis 30, und insbesondere 5 bis 25. In einer weiteren bevorzugten Ausführungsform sind R² und R³ Ethylen, Ethylen und Propylen, Ethylen und Butylen, oder Ethylen, Propylen und Butylen und die Summe aus n und m ist 6 bis 9, insbesondere 6,5 bis 8,5 und insbesondere 6,9 bis 7,9. In einer weiteren bevorzugten Ausführungsform sind R² und R³ Ethylen, Ethylen und Propylen, Ethylen und Butylen, oder Ethylen, Propylen und Butylen und die Summe aus n und m ist 11 bis 40, insbesondere 12 bis 30 und insbesondere 13,5 bis 25. In einer besonders bevorzugten Ausführungsform sind R¹ und R² Ethylen, Ethylen und Propylen, Ethylen und Butylen, oder Ethylen, Propylen und Butylen und die Summe aus n und m ist 6 bis 14, insbesondere 8 bis 12 und insbesondere 9 bis 11.

Beim Aminalkoxylat (A) sind besonders bevorzugt R² und R³ Ethylen, und die Summe aus n und m ist 2 bis 60, bevorzugt 2 bis 40, besonders bevorzugt 3 bis 30, und insbesondere 5 bis 25. In einer weiteren besonders bevorzugten Ausführungsform sind R² und R³ Ethylen und die Summe aus n und m ist 6 bis 9, insbesondere 6,5 bis 8,5 und insbesondere 6,9 bis 7,9. In einer weiteren besonders bevorzugten Ausführungsform sind R² und R³ Ethylen und die Summe aus n und m ist 11 bis 40, insbesondere 12 bis 30 und insbesondere 13,5 bis 25.

Die Verbindungen (A) und (AQ) können als Gemische von Stereoisomeren vorliegen oder als isolierte Stereoisomere. Tautomere und Betaine sind von den Strukturen (A) und (AQ) ebenfalls umfasst.

Die erfindungsgemäße Zusammensetzung enthält meist 0,1 bis 90 Gew.% des Alkoxylats, bevorzugt 1 bis 50 Gew.% und insbesondere 3 bis 30 Gew.%.

Die erfindungsgemäßen Zusammensetzungen umfassen ein Pestizid mit mindestens einer H-aciden Gruppe oder dessen anionisches Salz sowie optional ein oder mehrere weitere Pestizide.

Der Begriff Pestizide bezeichnet mindestens einen Wirkstoff ausgewählt aus der Gruppe der Fungizide, Insektizide, Nematizide, Herbizide, Safener, Molluskizide, Rodentizide und/oder Wachstumsregulatoren. Bevorzugte Pestizide sind Fungizide, Insektizide, Herbizide, und Wachstumsregulatoren. Besonders bevorzugte Pestizide sind Herbizide und Wachstumsregulatoren. Auch Mischungen von Pestiziden aus zwei oder mehr der vorgenannten Klassen können verwendet werden. Der Fachmann ist vertraut mit solchen Pestiziden, die beispielsweise in Pesticide Manual, 14th Ed. (2006), The British Crop Protection Council, London, gefunden werden können. Geeignete Pestizide sind:
A) Strobilurine:
   Azoxystrobin, Dimoxystrobin, Coumoxystrobin, Coumethoxystrobin, Enestroburin, Fluoxastrobin, Kresoxim-methyl, Metominostrobin, Orysastrobin, Picoxystrobin, Pyraclostrobin, Pyrametostrobin, Pyraoxystrobin, Pyribencarb, Trifloxystrobin, 2-[2-(2,5-Dimethylphenyl-oxymethyl)phenyl]-3-methoxy-acrylsäuremethylester, 2-(2-(3-(2,6-dichlorphenyl)-1-methyl-allylideneaminooxymethyl)-phenyl)-2-methoxyimino-N-methyl-acetamide;
B) Carbonsäureamide:
   - Carbonsäureanilide: Benalaxyl, Benalaxyl-M, Benodanil, Bixafen, Boscalid, Carboxin, Fenfuram, Fenhexamid, Flutolanil, Furametpyr, Isopyrazam, Isotianil, Kiralaxyl, Mepronil, Metalaxyl, Metalaxyl-M (Mefenoxam), Ofurace, Oxadixyl, Oxycarboxin, Penflufen (N-(2-(1,3-Dimethyl-butyl)-phenyl)-1,3-dimethyl-5-fluor-1H-pyrazol-4-carboxamid), Penthiopyrad, Sedaxane, Tecloftalam, Thifluzamide, Tiadinil, 2-Amino-4-methyl-thiazol-5-carboxanilid, N-(3',4',5'-Trifluorbiphenyl-2-yl)-3-difluormethyl-1-methyl-1 H-pyrazol-4-carboxamid, N-(4'-Trifluormethylthiobiphenyl-2-yl)-3-difluormethyl-1-methyl-1 H-pyrazol-4-carboxamid, N-(2-(1,3,3-Trimethyl-butyl)-phenyl)-1,3-dimethyl-5-fluor-1H-pyrazol-4-carboxamid;
   - Carbonsäuremorpholide: Dimethomorph, Flumorph, Pyrimorph;
   - Benzoesäureamide: Flumetover, Fluopicolide, Fluopyram, Zoxamid;
   - Sonstige Carbonsäureamide: Carpropamid, Diclocymet, Mandipropamid, Oxytetracyclin, Silthiofam, N-(6-methoxy-pyridin-3-yl)cyclopropancarbonsäureamid;
C) Azole:
   - Triazoie: Azaconazol, Bitertanol, Bromuconazol, Cyproconazol, Difenoconazol, Diniconazol, Diniconazol-M, Epoxiconazol, Fenbuconazol, Fluquinconazol, Flusilazol, Flutriafol, Hexaconazol, Imibenconazol, Ipconazol, Metconazol, Myclobutanil, Oxpoconazol, Paclobutrazol, Penconazol, Propiconazol, Prothioconazol, Simeconazol, Tebuconazol, Tetraconazol, Triadimefon, Triadimenol, Triticonazol, Uniconazol;
   - Imidazole: Cyazofamid, Imazalil, Imazalilsulfat, Pefurazoat, Prochloraz, Triflumizol;
   - Benzimidazole: Benomyl, Carbendazim, Fuberidazole, Thiabendazol;
   - Sonstige: Ethaboxam, Etridiazol, Hymexazol, 2-(4-Chlor-phenyl)-N-[4-(3,4-dimethoxy-phenyl)-isoxazol-5-yl]-2-prop-2-inyloxy-acetamid;
D) Stickstoffhaltige Heterocyclylverbindungen
   - Pyridine: Fluazinam, Pyrifenox, 3-[5-(4-Chfor-phenyl)-2,3-dimethyl-isaxazolidin-3-yl]-pyridin, 3-[5-(4-Methyl-phenyl)-2,3-dimethyl-isoxazolidin-3-yl]-pyridin;
   - Pyrimidine: Bupirimat, Cyprodinil, Diflumetorim, Fenarimol, Ferimzone, Mepanipyrim, Nitrapyrin, Nuarimol, Pyrimethanil;
   - Piperazine: Triforine;
   - Pyrrole: Fludioxonil, Fenpiclonil;
   - Morpholine: Aldimorph, Dodemorph, Dodemorphacetat, Fenpropimorph, Tridemorph;
   - Piperidine: Fenpropidin;
   - Dicarboximide: Fluorimid, Iprodione, Procymidone, Vinclozolin;
   - nichtaromatische 5-Ring-Heterocyclen: Famoxadon, Fenamidon, Flutianil, Octhilinon, Probenazol, 5-Amino-2-isopropyl-3-oxo-4-ortho-tolyl-2,3-dihydropyrazol-1-thiocarbonsäureS-allylester;
   - sonstige: Acibenzolar-S-methyl, Amisulbrom, Anilazin, Blasticidin-S, Captafol, Captan, Chinomethionat, Dazomet, Debacarb, Diclomezine, Difenzoquat, Difenzoquatmethylsulfat, Fenoxanil, Folpet, Oxolinsäure, Piperafin, Proquinazid, Pyroquilon, Quinoxyfen, Triazoxid, Tricyclazol, 2-Butoxy-6-jod-3-propyl-chromen-4-on, 5-Chlor-1-(4,6-dimethoxy-pyrimidin-2-yl)-2-methyl-1H-benzoimidazol, 5-Chlor-7-(4-methylpiperidin-1-yl)-6-(2,4,6-trifluor-phenyl)-[1,2,4]triazolo[1,5-a]pyrimidin, 5-Ethyl-6-octyl-[1,2,4]triazolo[1,5-a]pyrimidin-7-ylamin;
E) Carbamate und Dithiocarbamate
   - Thio- und Dithiocarbamate: Ferbam, Mancozeb, Maneb, Metam, Methasulphocarb, Metiram, Propineb, Thiram, Zineb, Ziram;
   - Carbamate: Diethofencarb, Benthiavalicarb, Iprovalicarb, Propamocarb, Propamocarb-hydrochlorid, Valiphenal, N-(1-(1-(4-Cyanophenyl)ethansulfonyl)-but-2-yl)carbaminsäure-(4-fluorphenyl)ester;
F) Sonstige Fungizide
   - Guanidine: Dodine, Dodine freie Base, Guazatin, Guazatinacetat, Iminoctadin, Iminoctadin-triacetat, Iminoctadin-tris(albesilat);
   - Antibiotika: Kasugamycin, Kasugamycinhydrochlorid-Mydrat, Polyoxine, Streptomycin, Validamycin A;
   - Nitrophenylderivate: Binapacryl, Dicloran, Dinobuton, Dinocap, Nitrothal-Isopropyl, Tecnazen;
   - Organometallverbindungen: Fentin-Salze wie beispielsweise Fentin-acetat, Fentinchlorid, Fentin-hydroxid;
   - Schwefelhaltige Heterocyclylverbindungen: Dithianon, Isoprothiolane;
   - Organophosphorverbindungen: Edifenphos, Fosetyl, Fosetyl-Aluminium, Iprobenfos, Phosphorige Säure und ihre Salze, Pyrazophos, Tolclofos-methyl;
   - Organochlorverbindungen: Chlorthalonil, Dichlofluanid, Dichlorphen, Flusulfamide, Hexachlorbenzol, Pencycuron, Pentachlorphenol und dessen Salze, Phthalid, Quintozen, Thiophanat-Methyl, Tolylfluanid, N-(4-Chlor-2-nitro-phenyl)-N-ethyl-4-methyl-benzolsulfonamid;
   - Anorganische Wirkstoffe: Phosphorige Säure und ihre Salze, Bordeaux Brühe, Kupfersalze wie beispielsweise Kupferacetat, Kupferhydroxid, Kupferoxychlorid, basisches Kupfersulfat, Schwefel;
   - Biologische Pilzbekämpfungsmittel, Pflanzenstärkungsmittel: Bacillus subtilis-Stamm NRRL-Nr. B-21661 (z.B. die Produkte RHAPSODY^{®}, SERENADE^{®} MAX und SERENADE^{®} ASO der Fa. AgraQuest, Inc., USA.), Bacillus pumilus-Stamm NRRL-Nr. B-30087 (z.B. SONATA^{®} and BALLAD^{®} Plus der Fa. AgraQuest, Inc., USA), Ulocladium oudemansii (z.B. BOTRY-ZEN der Fa. BotriZen Ltd., Neuseeland), Chitosan (z.B. ARMOUR-ZEN der Fa. BotriZen Ltd., Neuseeland).
   - Sonstige: Biphenyl, Bronopol, Cyflufenamid, Cymoxanil, Diphenylamin, Metrafenon, Mildiomycin, Oxin-Kupfer, Prohexadion-Calcium, Spiroxamin, Tolylfluanid, N-(Cyclopropylmethoxyimino-(6-difluormethoxy-2,3-difluor-phenyl)-methyl)-2-phenylacetamid, N'-(4-(4-Chlor-3-trifluormethyl-phenoxy)-2,5-dimethyl-phenyl)-N-ethyl-N-methylformamidin, N'-(4-(4-Fluor-3-trifluormethyl-phenoxy)-2,5-dimethyl-phenyl)-N-ethyl-N-methylformamidin, N'-(2-Methyl-5-trifluormethyl-4-(3-trimethylsilanyl-propoxy)-phenyl)-N-ethyl-N-methylformamidin, N'-(5-Difluormethyl-2-methyl-4-(3-frimethylsilanylpropoxy)-phenyl)-N-ethyl-N-methylformamidin, 2-{1-[2-(5-Methyl-3-trifluormethylpyrazol-1-yl)-acetyl]-piperidin-4-yl}-thiazol-4-carboxylsäure-methyl-(1,2,3,4-tetrahydronaphthalen-1-yl}-amid, 2-{1-(2-(5-Methyl-3-trifluormethyl-pyrazol-1-yl)-acetyl]-piperidin-4-yl}-thiazol-4-carboxylsäure-methyl-(R)-1,2,3,4-tetrahydronaphthalen-1-ylamid, Essigsäure-6-tert.-butyl-8-fluor-2,3-dimethyl-quinolin-4-yl-ester, Methoxy-essigsäure-6-tert.-butyl-8-fluor-2,3-dimethyl-quinolin-4-yl-ester, N-Methyl-2-{1-[2-(5-methyl-3-trifluormethyl-1 H-pyrazol-1-yl)-acetyl]-piperidin-4-yl}-N-[(1R)-1,2,3,4-tetrahydronaphthalen-1-yl]-4-thiazolcarboxamid;
G) Wachstumsregler
   Abscisinsäure, Amidochlor, Ancymidol , 6-Benzylaminopurin, Brassinolid, Butralin, Chlormequat (Chlormequatchlorid), Cholinchlorid, Cyclanilid, Daminozid, Dikegulac, Dimethipin, 2,6-Dimethylpuridin, Ethephon, Flumetralin, Flurprimidol, Fluthiacet, Forchlorfenuron, Gibbereilinsäure, Inabenfid, Indol-3-essigsäure, Maleinsäurehydrazid, Mefluidid, Mepiquat (Mepiquatchlorid), Metconazol, Naphthalenessigsäure, N-6-Benzyladenin, Paclobutrazol, Prohexadion (Prohexadion-Calcium), Prohydrojasmon, Thidiazuron, Triapenthenol, Tributylphosphorotrithioat, 2,3,5-tri-Jodbenzoesäure, Trinexapac-ethyl und Uniconazol;
H) Herbizide
   - Acetamide: Acetochlor, Alachlor, Butachlor, Dimethachlor, Dimethenamid, Flufenacet, Mefenacet, Metolachlor, Metazachlor, Napropamid, Naproanilid, Pethoxamid, Pretilachlor, Propachlor, Thenylchlor;
   - Aminosäureanaloga: Bilanafos, Glyphosat, Glufosinat, Sulfosat;
   - Aryloxyphenoxypropionate: Clodinafop, Cyhalofop-butyl, Fenoxaprop, Fluazifop, Haloxyfop, Metamifop, Propaquizafop, Quizalofop, Quizalofop-P-tefuryl;
   - Bipyridyle: Diquat, Paraquat;
   - Carbamate und Thiocarbamate: Asulam, Butylate, Carbetamide, Desmedipham, Dimepiperat, Eptam (EPTC), Esprocarb, Molinate, Orbencarb, Phenmedipham, Prosulfocarb, Pyributicarb, Thiobencarb, Triallate;
   - Cyclohexanedione: Butroxydim, Clethodim, Cycloxydim, Profoxydim, Sethoxydim, Tepraloxydim, Tralkoxydim;
   - Dinitroaniline: Benfluralin, Ethalfluralin, Oryzalin, Pendimethalin, Prodiamine, Trifluralin;
   - Diphenylether: Acifluorfen, Aclonifen, Bifenox, Diclofop, Ethoxyfen, Fomesafen, Lactofen, Oxyfluorfen;
   - Hydroxybenzonitrile: Bromoxynil, Dichlobenil, loxynil;
   - Imidazolinone: Imazamethabenz, Imazamox, Imazapic, Imazapyr, Imazaquin, Imazethapyr;
   - Phenoxyessigsäuren: Clomeprop, 2,4-Dichlorphenoxyessigsäure (2,4-D), 2,4-DB, Dichlorprop, MCPA, MCPA-thioethyl, MCPB, Mecoprop;
   - Pyrazine: Chloridazon, Flufenpyr-ethyl, Fluthiacet, Norflurazon, Pyridat;
   - Pyridine: Arrtinopyralid, Clopyralid, Diflufenican, Dithiopyr, Fluridone, Fluroxypyr, Picloram, Picolinafen, Thiazopyr;
   - Sulfonylharnstoffe: Amidosulfuron, Azimsulfuron, Bensulfuron, Chlorimuron-Ethyl, Chlorsulfuron, Cinosulfuron, Cyclosulfamuron, Ethoxysulfuron, Flazasulfuron, Flucetosulfuron, Flupyrsulfuron, Foramsulfuron, Halosulfuron, Imazosulfuron, lodosulfuron, Mesosulfuron, Metsulfuron-methyl, Nicosulfuron, Oxasulfuron, Primisulfuron, Prosulfuron, Pyrazosulfuron, Rimsulfuron, Sulfometuron, Sulfosulfuron, Thifensulfuron, Triasulfuron, Tribenuron, Trifloxysulfuron, Triflusulfuron, Tritosulfuron, 1-((2-Chlor-6-propyl-imidazo[1,2-b]pyridazin-3-yl)sulfonyl)-3-(4,6-dimethoxy-pyrimidin-2-yl)harnstoff;
   - Triazine: Ametryn, Atrazin, Cyanazin, Dimethametryn, Ethiozin, Hexazinon, Metamitron, Metribtizin, Prometryn, Simazin, Terbuthylazin, Terbutryn, Triaziflam;
   - Harnstoffe: Chlorotoluron, Daimuron, Diuron, Fluometuron, Isoproturon, Linuron, Methabenzthiazuron,Tebuthiuron;
   - andere Hemmstoffe der Acetolactatsynthase: Bispyribac-Natrium, Cloransulam-Methyl, Diclosulam, Florasulam, Flucarbazone, Flumetsulam, Metosulam, Orthosulfamuron, Penoxsulam, Propoxycarbazone, Pyribambenz-Propyl, Pyribenzoxim, Pyriftalid, Pyriminobac-methyl, Pyrimisulfan, Pyrithiobac, Pyroxasulfon, Pyroxsulam;
   - Sonstige: Amicarbazon, Aminotriazol, Anilofos, Beflubutamid, Benazolin, Bencarbazon, Benfluresat, Benzofenap, Bentazon, Benzobicyclon, Bromacil, Bromobutid, Butafenacil, Butamifos, Cafenstrole, Carfentrazone, Cinidon-Ethlyl, Chlorthal, Cinmethylin, Clomazone, Cumyluron, Cyprosulfamid, Dicamba, Difenzoquat, Diflufenzopyr, Drechslera monoceras, Endothal, Ethofumesat, Etobenzanid, Fentrazamide, Flumiclorac-Pentyl, Flumioxazin, Flupoxam, Fluorochloridon, Flurtamon, Indanofan, Isoxaben, Isoxaflutol, Lenacil, Propanil, Propyzamid, Quinclorac, Quinmerac, Mesotrion, Methylarsensäure, Naptalam, Oxadiargyl, Oxadiazon, Oxaziclomefon, Pentoxazon, Pinoxaden, Pyraclonil, Pyraflufen-Ethyl, Pyrasulfotol, Pyrazoxyfen, Pyrazolynat, Quinoclamin, Saflufenacil, Sulcotrion, Sulfentrazon, Terbacil, Tefuryltrion, Tembotrion, Thiencarbazon, Topramezon, 4-Hydroxy-3-[2-(2-methoxy-ethoxymethyl)-6-trifluormethyl-pyridin-3-carbonyl]-bicyclo[3.2.1]oct-3-en-2-on, (3-[2-Chlor-4-fluor-5-(3-methyl-2,6-dioxo-4-trifluormethyl-3,6-dihydro-2H-pyrimidin-1-yl)-phenoxy]-pyridin-2-yloxy)-essigsäureethylester, 6-Amino-5-chlor-2-cyclopropylpyrimidin-4-carboxylsäuremethylester, 6-Chlor-3-(2-cyclopropyl-6-methyl-phenoxy)-pyridazin-4-ol, 4-Amino-3-chlor-6-(4-chlor-phenyl)-5-fluor-pyridin-2-carboxylsäure, 4-Amino-3-chlor-6-(4-chlor-2-fluor-3-methoxy-phenyl)-pyridin-2-carboxylsäuremethylester und 4-Amino-3-chlor-6-(4-chloro-3-dimethylamino-2-fluor-phenyl)-pyridin-2-carboxylsäuremethylester;
I) Insektizide
   - Organo(thio)phosphate: Acephat, Azamethiphos, Azinphos-methyl, Chlorpyrifos, Chlorpyrifos-Methyl, Chlorfenvinphos, Diazinon, Dichlorvos, Dicrotophos, Dimethoat, Disulfoton, Ethion, Fenitrothion, Fenthion, Isoxathion, Malathion, Methamidophos, Methidathion, Methyl-Parathion, Mevinphos, Monocrotophos, Oxydemeton-Methyl, Paraoxon, Parathion, Phenthoate, Phosalone, Phosmet, Phosphamidon, Phorate, Phoxim, Pirimiphos-Methyl, Profenofos, Prothiofos, Sulprophos, Tetrachlorvinphos, Terbufos, Triazophos, Trichlorfon;
   - Carbamate: Alanycarb, Aldicarb, Bendiocarb, Benfuracarb, Carbaryl, Carbofuran, Carbosulfan, Fenoxycarb, Furathiocarb, Methiocarb, Methomyl, Oxamyl, Pirimicarb, Propoxur, Thiodicarb, Triazamate;
   - Pyrethroide: Allethrin, Bifenthrin, Cyfluthrin, Cyhalothrin, Cyphenothrin, Cypermethrin, alpha-Cypermethrin, beta-Cypermethrin, zeta-Cypermethrin, Deltamethrin, Esfenvalerat, Etofenprox, Fenpropathrin, Fenvalerate, Imiprothrin, Lambda-Cyhalothrin, Permethrin, Prallethrin, Pyrethrin I und II, Resmethrin, Silafluofen, tau-Fluvalinat, Tefluthrin, Tetramethrin, Tralomethrin, Transfluthrin, Profluthrin, Dimefluthrin,
   - Hemmstoffe des Insektenwachsturns: a) Chitinsynthese-Hemmstoffe: Benzoylharnstoffe: Chlorfluazuron, Cyramazin, Diflubenzuron, Flucycloxuron, Flufenoxuron, Hexaflumuron, Lufenuron, Novaluron, Teflubenzuron, Triflumuron; Buprofezin, Diofenolan, Hexythiazox, Etoxazol, Clofentazin; b) Ecdyson-Antagonisten: Halofenozid, Methoxyfenozid, Tebufenozid, Azadirachtin; c) Juvenoide: Pyriproxyfen, Methoprene, Fenoxycarb; d) Lipidbiosynthese-Hemmstoffe: Spirodiclofen, Spiromesifen, Spirotetramat;
   - Nikotinreceptor-Agonisten/Antagonisten: Clothianidin, Dinotefuran, Imidacloprid, Thiamethoxam, Nitenpyram, Acetamiprid, Thiacloprid, 1-(2-chloro-thiazol-5-yl-methyl)-2-nitrimino-3,5-dimethyl-[1,3,5]triazinan;
   - GABA-Antagonisten: Endosulfan, Ethiprol, Fipronil, Vaniliprol, Pyrafluprol, Pyriprol, 5-Amino-1-(2,6-dichlor-4-methyl-phenyl)-4-sulfinamoyl-pyrazol-3-thiocarbonsäureamid;
   - Macrocyclische Lactone: Abamectin, Emamectin, Milbemectin, Lepimectin, Spinosad, Spinetoram;
   - Mitochondriale Elektronentransportketten-Inhibitor (MET!)! I Akarizide: Fenazaquin, Pyridaben, Tebufenpyrad, Tolfenpyrad, Flufenerim;
   - METI II und f)) Substanzen: Acequinocyl, Fluacyprim, Hydramethylnon;
   - Entkoppler: Chlorfenapyr;
   - Hemmstoffe der oxidativen Phosphorylierung: Cyhexatin, Diafenthiuron, Fenbutatinoxid, Propargit;
   - Hemmstoffe der Häutung der Insekten: Cryomazin;
   - Hemmstoffe von ,mixed function oxidases': Piperonylbutoxid;
   - Natriumkanalblocker: Indoxacarb, Metaflumizon;
   - Sonstige: Benclothiaz, Bifenazate, Cartap, Flonicamid, Pyridalyl, Pymetrozin, Schwefel, Thiocyclam, Flubendiamid, Chlorantraniliprol, Cyazypyr (HGW86); Cyenopyrafen, Flupyrazofos, Cyflumetofen, Amidoflumet, Imicyafos, Bistrifluron und Pyrifluquinazon.

Die erfindungsgemäßen Zusammensetzungen umfassen mindestens ein Pestizid mit mindestens einer H-aciden Gruppe (wie Carbonsäuregruppe, Phosphonsäuregruppe, Phosphinsäuregruppe) oder deren anionische Salze (z. B. Mono-, Di-, oder Trisalze). Diese anionischen Salze der Pestizide mit einer H-aciden Gruppe sind auch als anionische Pestizide in der Gruppe A- geeignet. Bevorzugte Pestizide mit einer H-aciden Gruppe sind Herbizide mit einer H-aciden Gruppe. Beispiele für Herbizide mit einer H-aciden Gruppen sind Aminosäureanaloga (wie Glyphosate oder Glufosinate) oder Imidazolinone (wie Imazamethabenz, Imazamox, Imazapic, Imazapyr, Imazaquin, Imazethapyr).
Besonders bevorzugte Pestizide mit einer H-aciden gruppen sind Glyphosat und Glufosinat. In einer weiteren bevorzugten Ausführungsform sind Pestizide mit einer H-aciden Gruppe Imidazolinone.

Besonders bevorzugt umfassen die Zusammensetzungen ein Pestizid mit einer H-aciden Gruppe und ein weiteres Pestizid. In einer weiteren Ausführungsform umfassen die Zusammensetzungen Mischungen von mindestens zwei Pestiziden mit einer H-aciden Gruppe, und optional weitere Pestizide (wie mindestens ein Fungizid, Herbizid, Insektizid, und/oder Safener, wobei Fungizide und/oder Herbizide bevorzugt sind).

In einer weiteren bevorzugten Ausführungsform umfassen die Zusammensetzungen Glyphosat (z.B. als freie Säure, Natriumsalz, Sesquinatriumsalz, Kaliumsalz, Dikaliumsalz, Ammoniumsalz, Diammoniumsalz, Dimethylammoniumsalz, Trimesiumsalz oder Isopropylaminsalz) oder Glufosinat (z.B. als Ammoniumsalz). Besonders bevorzugt umfassen die Zusammensetzungen Glyphosat (z.B. als Kaliumsalz, Ammoniumsalz, Isopropylaminsalz). Besonders bevorzugt umfassen die Zusammensetzungen Glyphosat oder Glufosinat, und zusätzlich ein weiteres Herbizid. In einer weiteren bevorzugten Ausführungsform umfassen die Zusammensetzungen Glyphosat oder Glufosinat, und zusätzlich ein weiteres Pestizid (wie mindestens ein Fungizid, Herbizid, Insektizid, und/oder Safener, wobei Fungizide und/oder Herbizide bevorzugt sind).

Die erfindungsgemäßen Zusammensetzungen können weiterhin auch für agrochemische Formulierungen übliche Hilfsmittel enthalten, wobei sich die Wahl der Hilfsmittel nach der konkreten Anwendungsform, dem Formulierungstyp bzw. dem Wirkstoff richtet. Beispiele für geeignete Hilfsmittel sind Lösungsmittel, feste Trägerstoffe, oberflächenaktive Stoffe (wie Tenside, Solubilisatoren, Schutzkolloide, Netzmittel und Haftmittel), organische und anorganische Verdicker, Bakterizide, Frostschutzmittel, Entschäumer, ggf. Farbstoffe und Kleber (z. B. für Saatgutbehandlung) oder übliche Hilfsmittel für Köderformulierung (z. B. Lockstoffe, Futtermittel, Bitterstoffe).

Als Lösungsmittel kommen Wasser oder organische Lösungsmittel wie Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Gykole, Ketone wie Cyclohexanon, gamma-Butyrolacton, Dimethylfettsäureamide, Fettsäuren und Fettsäureester und stark polare Lösungsmittel, z.B. Amine wie N-Methylpyrrolidon, in Betracht. Grundsätzlich können auch Lösungsmittelgemische verwendet werden sowie Gemische aus den vorstehend genannten Lösungsmitteln und Wasser.

Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.
Als oberflächenaktive Stoffe (Adjuvantien, Netz-, Haft-, Dispergier- oder Emulgiermittel) kommen die Alkali-, Erdalkali-, Ammoniumsalze von aromatischen Sulfonsäuren, z. B. von Lignin-(Borresperse^{®}-Typen, Borregaard, Norwegen), Phenol-, Naphthalin-(Morwet^{®}-Typen, Akzo Nobel, USA) und Dibutylnaphthalinsulfonsäure (Nekal^{®}-Typen, BASF, Deutschland), sowie von Fettsäuren, Alkyl- und Alkylarylsulfonaten, Alkyl-, Laurylether- und Fettalkoholsulfaten, sowie Salze sulfatierter Hexa-, Hepta- und Octadecanole sowie von Fettalkoholglykolethern, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen sowie Proteine, denaturierte Proteine, Polysaccharide (z.B. Methylcellulose), hydrophob modifizierte Stärken, Polyvinylalkohol (Mowiol^{®}-Typen, Clariant, Schweiz), Polycarboxylate (Sokalan^{®}-Typen, BASF, Deutschland), Polyalkoxylate, Polyvinylamin (Lupamin^{®}-Typen, BASF, Deutschland), Poiyethylenimin (Lupasol^{®}-Typen, BASF, Deutschland), Polyvinylpyrrolidon und deren Copolymere in Betracht.

Die erfindungsgemäße Zusammensetzung kann 0,1 bis 40 Gew.%, bevorzugt 1 bis 30 und insbesondere 2 bis 20 Gew.% oberflächenaktive Stoffe umfassen, wobei die Menge des Alkoxlylats (A) und (AQ) nicht mit einberechnet werden.

Geeignete Verdicker sind Verbindungen, die der Formulierung ein modifiziertes Fließverhalten verleihen, d. h. eine hohe Viskosität im Ruhezustand und niedrige Viskosität im bewegten Zustand. Beispiele sind Polysaccharide, Proteine (wie Casein oder gelatine), synthetische Polymere, oder anorganische Schichtmineralien. Solche Verdicker sind kommerziell erhältlich, beispielsweise Xanthan Gum (Kelzan^{®}, CP Kelco, USA), Rhodopol^{®} 23 (Rhodia, Frankreich) oder Veegum^{®} (R.T. Vanderbilt, USA) oder Attaclay^{®} (Engelhard Corp., NJ, USA). Der Gehalt an Verdicker in der Formulierung richtet sich nach der Wirksamkeit des Verdickers. Der Fachmann wird einen Gehalt wählen, um die gewünschte Viskosität der Formulierung zu erhalten. Der Gehalt wird meist 0,01 bis 10 Ges.% betragen.

Bakterizide können zur Stabilisierung der Zusammensetzung zugesetzt werden. Beispiele für Bakterizide sind solche basierend auf Diclorophen und Benzylalkoholhemiformal sowie Isothiazolinonderivaten wie Alkylisothiazolinonen und Benzisothiazolinonen (Acticide^{®} MBS der Fa. Thor Chemie). Beispiele für geeignete Frostschutzmittel sind Ethylenglycol, Propylenglycol, Harnstoff und Glycerin. Beispiele für Entschäumer sind Silikonemulsionen (wie z. B. Silikon^{®} SRE, Wacker, Deutschland oder Rhodorsil^{®}, Rhodia, Frankreich), langkettige Alkohole, Fettsäuren, Salze von Fettsäuren, fluororganische Verbindungen und deren Gemische.

Die erfindungsgemäße Zusammensetzung kann bevorzugt in Form einer agroschemischen Formulierung vorliegen. Beispiele für solche Formulierungen und ihrer Herstellung sind:
i) Wasserlösliche Konzentrate (SL, LS): 10 Gew.-Teile der Wirkstoffe werden mit 90 Gew.-Teilen Wasser oder einem wasserlöslichen Lösungsmittel gelöst. Alternativ werden Netzmittel oder andere Hilfsmittel zugefügt. Bei der Verdünnung in Wasser löst sich der Wirkstoff. Man erhält auf diese Weise eine Zusammensetzung mit 10 Gew.-% Wirkstoffgehalt.
ii) Dispergierbare Konzentrate (DC): 20 Gew.-Teile der Wirkstoffe werden in 70 Gew.-Teilen Cyclohexanon unter Zusatz von 10 Gew.-Teilen eines Dispergiermittels z. B. Polyvinylpyrrolidon gelöst. Bei Verdünnung in Wasser ergibt sich eine Dispersion. Der Wirkstoffgehalt beträgt 20 Gew.-%
iii) Emulgierbare Konzentrate (EC): 15 Gew.-Teile der Wirkstoffe werden in 75 Gew.-Teilen Xylol unter Zusatz von Ca-Dodecylbenzolsulfonat und Ricinusölethoxylat (jeweils 5 Gew.-Teile) gelöst. Bei der Verdünnung in Wasser ergibt sich eine Emulsion. Die Zusammensetzung hat 15 Gew.-% Wirkstoffgehalt.
iv) Emulsionen (EW, EO, ES): 25 Gew.-Teile der Wirkstoffe werden in 35 Gew.-Teile Xylol unter Zusatz von Ca-Dodecylbenzolsulfonat und Ricinusölethoxylat (jeweils 5 Gew.-Teile) gelöst. Diese Mischung wird mittels einer Emulgiermaschine (z. B. Ultra-Turrax) in 30 Gew.-Teile Wasser gegeben und zu einer homogenen Emulsion gebracht. Bei der Verdünnung in Wasser ergibt sich eine Emulsion. Die Zusammensetzung hat einen Wirkstoffgehalt von 25 Ges.-%.
v) Suspensionen (SC, OD, FS): 20 Gew.-Teile der Wirkstoffe werden unter Zusatz von 10 Gew.-Teilen Dispergier- und Netzmitteln und 70 Gew.-Teilen Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs. Der Wirkstoffgehalt in der Zusammensetzung beträgt 20 Gew.-%.
vi) Wasserdispergierbare und wasserlösliche Granulate (WG, SG): 50 Gew.-Teile der Wirkstoffe werden unter Zusatz von 50 Gew.-Teilen Dispergier- und Netzmitteln fein gemahlen und mittels technischer Geräte (z. B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs. Die Zusammensetzung hat einen Wirkstoffgehalt von 50 Gew.-%.
vii) Wasserdispergierbare und wasserlösliche Pulver (WP, SP, SS, WS): 75 Gew.-Teile der Wirkstoffe werden unter Zusatz von 25 Gew.-Teilen Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs. Der Wirkstoffgehalt der Zusammensetzung beträgt 75 Gew.-%.
viii) Gele (GF): In einer Kugelmühle werden 20 Gew.-Teile der Wirkstoffe, 10 Gew.-Teile Dispergiermittel, 1 Gew.-Teil Quellmittel ("gelling agent") und 70 Gew.-Teile Wasser oder eines organischen Lösungsmittels zu einer feinen Suspension vermahlen. Bei der Verdünnung mit Wasser ergibt sich eine stabile Suspension mit 20 Gew.-% Wirkstoffgehalt.
ix) Stäube (DP, DS): 5 Gew.-Teile der Wirkstoffe werden fein gemahlen und mit 95 Gew.-Teilen feinteiligem Kaolin innig vermischt. Man erhält dadurch ein Stäubemittel mit 5 Gew.-% Wirkstoffgehalt.
x) Granulate (GR, FG, GG, MG): 0,5 Gew.-Teile der Wirkstoffe werden fein gemahlen und mit 99,5 Gewichtsteilen Trägerstoffe verbunden. Gängige Verfahren sind dabei die Extrusion, die Sprühtrocknung oder die Wirbelschicht. Man erhält dadurch ein Granulat für die Direktapplikation mit 0,5 Gew.-% Wirkstoffgehalt.
xi) ULV- Lösungen (UL): 10 Gew.-Teile der Wirkstoffe werden in 90 Gew.-Teilen eines organischen Lösungsmittel z. B. Xylol gelöst. Dadurch erhält man eine Zusammensetzung für die Direktapplikation mit 10 Gew.-% Wirkstoffgehalt.

Die Zusammensetzungen enthalten im allgemeinen 0,01 bis 95 Gew.-%, vorzugsweise 0,1 bis 90 Gew.-% der Pestizide.

Der Anwender verwendet die erfindungsgemäße Zusammensetzung üblicherweise für die Anwendung in einer Vordosiereinrichtung, im Rückenspritzer, im Spritztank oder im Sprühflugzeug. Dabei wird die Formulierung mit Wasser und/oder Puffer auf die gewünschte Anwendungskonzentration gebracht, wobei gegebenenfalls weitere Hilfsstoffe zugegeben werden, und so die anwendungsbereite Spritzbrühe (sog. Tankmix) erhalten wird. Üblicherweise werden 50 bis 500 Liter der anwendungsbereiten Spritzbrühe pro Hektar landwirtschaftlicher Nutzfläche aufgebracht, bevorzugt 100 bis 400 Liter. In speziellen Segmenten können diese Mengen auch über- (z.B. Obstbau) oder unterschritten (z.B. Flugzeugapplikation) werden. Die Wirkstoffkonzentrationen in den anwendungsfertigen Zubereitungen können in größeren Bereichen variiert werden. Im Allgemeinen liegen sie zwischen 0,0001 und 10%, vorzugsweise zwischen 0,01 und 1%.

Zu den Wirkstoffen oder den diese enhaltenden Zusammensetzungen können Öle verschiedenen Typs, Netzmittel, Driftreduktionsmittel, Sticker, Spreader, Adjuvantien, Dünger, Pflanzenstärkungsmittel, Spurenelemente, Herbizide, Bakterizide, Fungizide und/oder Schädlingsbekämpfungsmittel, gegebenenfalls auch erst unmittelbar vor der Anwendung, dem Tankmix zugesetzt werden. Diese Mittel können zu den erfindungsgemäßen Zusammensetzungen im Gewichtsverhältnis 1:100 bis 100:1, bevorzugt 1:10 bis 10:1 zugemischt werden. Als Adjuvanzien in diesem Sinne kommen insbesondere in Frage: organisch modifizierte Polysiloxane, z. B. Break Thru S 240^{®}; Alkoholalkoxylate, z. B. Atplus^{®} 245, Atplus^{®} MBA 1303, Plurafac^{®} LF 300 und Lutensol^{®} ON 30; EO-PO-Blockpolymerisate, z. B. Pluronic^{®} RPE 2035 und Genapol^{®} B; Alkoholethoxylate, z. B. Lutensol^{®} XP 80; und Natriumdioctylsulfosuccinat, z. B. Leophen^{®} RA.

Die Aufwandmengen des Wirkstoffs liegen bei der Anwendung im Pflanzenschutz je nach Art des gewünschten Effektes zwischen 0,001 und 2,0 kg Wirkstoff pro ha, bevorzugt zwischen 0,005 und 2 kg pro ha, besonders bevorzugt zwischen 0,05 und 0,9 kg pro ha, insbesondere zwischen 0,1 und 0,75 kg pro ha.

Die vorliegende Erfindung betrifft weiterhin ein Verfahren zur Bekämpfung von phytopathogenen Pilzen und/oder unerwünschtem Pflanzenwuchs und/oder unerwünschtem Insekten- oder Milbenbefall und/oder zur Regulation des Wachstums von Pflanzen, wobei man die erfindungsgemäße Zusammensetzung auf die jeweiligen Schädlinge, deren Lebensraum oder die vor dem jeweiligen Schädling zu schützenden Pflanzen, den Boden und/oder auf unerwünschte Pflanzen und/oder die Nutzpflanzen und/oder deren Lebensraum einwirken lässt.

Geeignete Nutzplanzen sind beispielsweise Getreide, z. B. Weizen, Roggen, Gerste, Triticale, Hafer oder Reis; Rüben, z. B. Zucker oder Futterrüben; Kern-, Stein und Beerenobst, z. B. Äpfel, Birnen, Pflaumen, Pfirsiche, Mandeln, Kirschen, Erdbeeren, Himbeeren, Johannisbeeren oder Stachelbeeren; Leguminosen, z. B. Bohnen, Linsen, Erbsen, Luzerne oder Soja; Ölpflanzen, z. B. Raps, Senf, Oliven, Sonnenblumen, Kokosnuss, Kakao, Rizinusbohnen, Ölpalme, Erdnüsse oder Soja; Kürbisgewächse, z. B. Kürbissse, Gurken oder Melonen; Faserpflanzen, z. B. Baumwolle, Flachs, Hanf oder Jute; Zitrusfrüchte, z. B. Orangen, Zitronen, Pampelmusen oder Mandarinen; Gemüsepflanzen, z. B. Spinat, Salat, Spargel, Kohlpflanzen, Möhren, Zwiebeln, Tomaten, Kartoffeln, Kürbis oder Paprika; Lorbeergewächse, z. B. Avocados, Zimt oder Kampher; Energie- und Rohstoffpflanzen, z. B. Mais, Soja, Weizen, Raps, Zuckerrohr oder Ölpalme; Mais; Tabak; Nüsse; Kaffee; Tee; Bananen; Wein (Tafel- und Weintrauben); Hopfen; Gras, z. B. Rasen; Süßkraut (Stevia rebaudania); Kautschukpflanzen; Zierund Forstpflanzen, z. B. Blumen, Sträucher, Laub- und Nadelbäume sowie Vermehrungsmaterial, z. B. Samen, und Erntegut dieser Pflanzen.

Der Begriff Nutzplanzen schließt auch solche Pflanzen ein, die durch Züchtung, Mutagenese oder gentechnische Methoden verändert wurden einschließlich der auf dem Markt oder in Entwicklung befindlichen biotechnologischen Agrarprodukte. Gentechnisch veränderte Pflanzen sind Pflanzen, deren genetisches Material in einer Weise verändert worden ist, wie sie unter natürlichen Bedingungen durch Kreuzen, Mutationen oder natürliche Rekombination (d.h. Neuzusammenstellung der Erbinformation) nicht vorkommt. Dabei werden in der Regel ein oder mehrere Gene in das Erbgut der Pflanze integriert, um die Eigenschaften der Pflanze zu verbessern. Derartige gentechnische Veränderungen umfassen auch posttranslationale Modifikationen von Proteinen, Oligo- oder Polypeptiden z.B. mittels Glykolsylierung oder Bindung von Polymeren wie z.B. prenylierter, acetylierter oder farnelysierter Reste oder PEG-Reste.

Beispielhaft seien Pflanzen genannt, die durch züchterische und gentechnische Maßnahmen eine Toleranz gegen bestimmter Herbizidklassen, wie Hydroxyphenylpyruvat-Dioxygenase (HPPD)-Inhibitoren, Acetolactat-Synthase (ALS)-Inhibitoren wie z. B. Sulfonylharnstoffe (EP-A 257 993, US 5,013,659) oder Imidazolinone (z. B. US 6,222,100, WO 01/82685, WO 00/26390, WO 97/41218, WO 98/02526, WO 98/02527, WO 04/106529, WO 05/20673, WO 03/14357, WO 03/13225, WO 03/14356, WO 04/16073), Enolpyruvylshikimat-3-Phosphat-Synthase (EPSPS)-Inhibitoren wie z. B. Glyphosat (siehe z. B. WO 92/00377), Glutaminsynthetase (GS)-Inhibitoren wie z. B. Glufosinat (siehe z. B. EP-A 242 236, EP-A 242 246) oder Oxynil-Herbizide (siehe z. B. US 5,559,024) erworben haben. Durch Züchtung und Mutagenese wurde z. B. Clearfield^{®}-Raps (BASF SE, Deutschland) erzeugt, der eine Toleranz gegen Imidazolinone, z. B. Imazamox, hat. Mit Hilfe gentechnischer Methoden wurden Kulturpflanzen, wie Soja, Baumwolle, Mais, Rüben und Raps, erzeugt, die resistent gegen Glyphosat oder Glufosinat sind, erzeugt, welche unter den Handelsnamen RoudupReady^{®} (Glyphosat-resistent, Monsanto, U.S.A.) und Liberty Link^{®} (Glufosinat-resistent, Bayer CropScience, Deutschland) erhältlich sind.

Weiterhin sind auch Pflanzen umfasst, die mit Hilfe gentechnischer Maßnahmen ein oder mehrere Toxine, z. B. solche aus dem Bakterienstamm Bacillus, produzieren. Toxine, die durch solche gentechnisch veränderten Pflanzen hergestellt werden, umfassen z. B. insektizide Proteine von Bacillus spp., insbesondere von B. thuringiensis, wie die Endotoxine Cry1Ab, Cry1Ac, Cry1F, Cry1Fa2, Cry2Ab, Cry3A, Cry3Bb1, Cry9c, Cry34Ab1 oder Cry35Ab1; oder vegetative insektizide Proteine (VIPs), z. B. VIP1, VIP2, VIP3, oder VIP3A; insektizide Proteine von Nematoden-kolonisierenden Bakterien, z. B. Photorhabdus spp. oder Xenorhabdus spp.; Toxine aus tierischen Organismen, z. B. Wepsen,-, Spinnen- oder Skorpionstoxine; pilzliche Toxine, z. B. aus Streptomyceten; pflanzliche Lektine, z. B. aus Erbse oder Gerste; Agglutinine; Proteinase-Inhibitoren, z. B. Trypsin-Inhibitoren, Serinprotease-Inhibitoren, Patatin, Cystatin oder Papain-Inhibitoren; Ribosomen-inaktivierende Proteine (RIPs), z. B. Ricin, Mais-RIP, Abrin, Luffin, Saporin oder Bryodin; Steroid-metabolisierende Enzyme, z. B. 3-Hydroxysteroid-Oxidase, Ecdysteroid-IDP-Glycosyl-Transferase, Cholesterinoxidase, Ecdyson-Inhibitoren oder HMG-CoA-Reduktase; lonenkanalblocker, z. B. Inhibitoren von Natrium- oder Calziumkanälen; Juvenilhormon-Esterase; Rezeptoren für das diuretischen Hormon (Helicokininrezeptoren); Stilbensynthase, Bibenzylsynthase, Chitinasen und Glucanasen. Diese Toxine können in den Pflanzen auch als Prätoxine, Hybridproteine, verkürzte oder anderweitig modfizierte Proteine produziert werden. Hybridproteine zeichnen sich durch eine neue Kombination von verschiedenen Proteindomänen aus (siehe z. B. WO 2002/015701). Weitere Besipiele für derartige Toxine oder gentechnisch veränderte Pflanzen, die diese Toxine produzieren, sind in EP-A 374 753, WO 93/07278, WO 95/34656, EP-A 427 529, EP-A 451 878, WO 03/18810 und WO 03/52073 offenbart. Die Methoden zur Herstellung dieser gentechnisch veränderten Pflanzen sind dem Fachmann bekannt und z. B. in den oben erwähnten Publikationen dargelegt. Zahlreiche der zuvor genannten Toxine verleihen den Pflanzen, die diese produzieren, eine Toleranz gegen Schädlinge aus allen taxonomischen Arthropodenklassen, insbesondere gegen Käfer (Coeleropta), Zweiflügler (Diptera) und Schmetterlinge (Lepidoptera) und gegen Nematoden (Nematoda). Gentechnisch veränderte Pflanzen, die ein oder mehrere Gene, die für insektizide Toxine kodieren, produzieren sind z. B. in den oben erwähnten Publikationen beschrieben und zum Teil kommerziell erhältlich, wie z. B. YieldGard^{®} (Maissorten, die das Toxin Cry1Ab produzieren), YieldGard^{®} Plus (Maissorten, die die Toxine Cry1Ab und Cry3Bb1 produzieren), Starlink^{®} (Maissorten, die das Toxin Cry9c produzieren), Herculex^{®} RW (Maissorten, die die Toxine Cry34Ab1, Cry35Ab1 und das Enzym Phosphinothricin-N-Acetyltransferase [PAT] produzieren); NUCOTN^{®} 33B (Baumwollsorten, die das Toxin Cry1Ac produzieren), Bollgard^{®} I (Baumwollsorten, die das Toxin Cry1Ac produzieren), Bollgard^{®} II (Baumwollsorten, die die Toxine Cry1Ac und Cry2Ab2 produzieren); VIPCOT^{®} (Baumwollsorten, die ein VIP-Toxin produzieren); NewLeaf^{®} (Kartoffelsorten, die das Toxin Cry3A produzieren); Bt-Xtra^{®}, NatureGard^{®}, KnockOut^{®}, BiteGard^{®}, Protecta^{®}, Bt11 (z. B. Agrisure^{®} CB) und Bt176 von Syngenta Seeds SAS, Frankreich, (Maissorten, die das Toxin Cry1Ab und das PAT-Enyzm produzieren), MIR604 von Syngenta Seeds SAS, Frankreich (Maissorten, die ein modifizierte Version des Toxins Cry3A produzieren, siehe hierzu WO 03/018810), MON 863 von Monsanto Europe S.A., Belgien (Maissorten, die das Toxin Cry3Bb1 produzieren), IPC 531 von Monsanto Europe S.A., Belgien (Baumwollsorten, die eine modifizierte Version des Toxins Cry1Ac produzieren) und 1507 von Pioneer Overseas Corporation, Belgien (Maissorten, die das Toxin Cry1F und das PAT-Enyzm produzieren).

Weiterhin sind auch Pflanzen umfasst, die mit Hilfe gentechnische Maßnahmen ein oder mehrere Proteine produzieren, die eine erhöhte Resistenz oder Widerstandfähigkeit gegen bakterielle, virale oder pilzliche Pathogene bewirken, wie z. B. sogenannte Pathogenesis-related-Proteine (PR-Proteine, siehe EP-A 0 392 225), Resistenzproteine (z. B. Kartoffelsorten, die zwei Resistenzgene gegen Phytophthora infestans aus der mexikanischen Wildkartoffel Solanum bulbocastanum produzieren) oder T4-Lysozym (z. B. Kartoffelsorten, die durch die Produktion diese Proteins resistent gegen Bakterien wie Erwinia amylvora ist).

Weiterhin sind auch Pflanzen umfasst, deren Produktivität mit Hilfe gentechnischer Methoden verbessert wurde, indem z. B. die Ertragsfähigkeit (z. B. Biomasse, Kornertrag, Stärke-, Öl- oder Proteingehalt), die Toleranz gegenüber Trockenheit, Salz oder anderen begrenzenden Umweltfaktoren oder die Widerstandsfähigkeit gegenüber Schädlingen und pilzlichen, bakteriellen und viralen Pathogenen gesteigert wird. Weiterhin sind auch Pflanzen umfasst, deren Inhaltsstoffe insbesondere zur Verbesserung der menschlichen oder tierischen Ernährung mit Hilfe gentechnischer Methoden verändert wurden, indem z. B. Ölpflanzen gesundheitsfördernde langkettige Omega-3-Fettsäuren oder einfach ungesättigte Omega-9-Fettsäuren (z. B. Nexera^{®}-Raps, DOW Agro Sciences, Kanada) produzieren.

Die vorliegende Erfindung betrifft auch Saatgut (wie Saaten oder andere pflanzliche Vermehrungsmaterialien) enthaltend die erfindungsgemäße Zusammensetzung. Pflanzliche Vermehrungsmaterialien können vorbeugend zusammen mit oder bereits vor der Aussaat bzw. zusammen mit oder bereits vor dem Umpflanzen mit der erfindungsgemäßen Zusammensetzung behandelt werden. Für die Behandlung von Saatgut werden üblicherweise wasserlösliche Konzentrate (LS), Suspensionen (FS), Stäube (DS), wasserdispergierbare und wasserlösliche Pulver (WS, SS), Emulsionen (ES), emulgierbare Konzentrate (EC) und Gele (GF) verwendet. Diese Zusammensetzungen können auf die Vermehrungsmaterialien, insbesondere Saatgut, unverdünnt oder, bevorzugt, verdünnt angewendet werden. Hierbei kann die entsprechende Zusammensetzung 2 bis 10fach verdünnt werden, so dass in den für die Beize zu verwendeten Zusammensetzungen 0,01 to 60% Gew.%, vorzugsweise 0,1 to 40% Gew. % Wirkstoff vorhanden sind. Die Anwendung kann vor oder während der Aussaat erfolgen. Die Behandlung von pflanzlichem Vermehrungsmaterial, insbesondere die Behandlung von Saatgut, sind dem Fachmann bekannt, und erfolgen durch Bestäuben, Beschichten, Pelletieren, Eintauchen oder Tränken des pflanzlichen Vermehrungsmaterial, wobei die Behandlung bevorzugt durch Pelletieren, Beschichten und Bestäuben oder durch Furchenbehandlung erfolgt, so dass z. B. eine vorzeitige Keimung des Saatguts verhindert wird. Für die Saatgutbehandlung werden bevorzugt Suspensionen verwendet. Üblicherweise enthalten solche Zusammensetzungen 1 bis 800 g/l Wirkstoff, 1 bis 200 g/l Tenside, 0 bis 200 g/l Frostschutzmittel, 0 bis 400 g/l Bindemittel, 0 bis 200 g/l Farbstoffe und Lösungsmittel, vorzugsweise Wasser.

Die vorliegende Erfindung betrifft weiterhin ein Alkoxylat, wobei das Alkoxylat ein Aminalkoxylat (A) 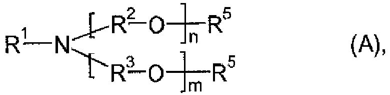 ist, wobei
- R¹: ein verzweigter, aliphatischer Alkylrest C₉H₁₉ ist,
- R², R³, und R⁷: unabhängig voneinander Ethylen, Propylen, Butylen oder eine Mischung davon sind,
- R⁵: ein H, C₁-C₆-Alkyl, C₂-C₆-Alkenyl, C₂-C₆-Alkinyl, -SO₃R^{a}, -P(O)OR^{b}OR^{c}, -CH₂CO₂R^{d}, oder -C(O)R^{e} ist,
- R⁶: ein C₁-C₆-Alkyl, C₂-C₆-Alkenyl, oder C₂-C₆-Alkinyl ist,
- R^{a} und R^{d}: unabhängig voneinander ein H, anorganische oder organische Kationen sind,
- R^{b} und R^{c}: unabhängig voneinander ein H, anorganische oder organische Kationen, C₁-C₆-Alkyl, C₂-C₆-Alkenyl oder C₂-C₆-Alkinyl sind,
- R^{e}: C₁-C₂₂-Alkyl, C₂-C₂₂-Alkenyl, C₂-C₂₂-Alkinyl, C₆-C₂₂-Aryl, C₇-C₂₂-Alkylaryl ist,
- n, m und p: unabhängig voneinander ein Wert von 1 bis 30 haben, und
- A-: ein landwirtschaftlich akzeptables Anion oder, falls R³ ein Sauerstoffanion ist, A-nicht vorhanden ist,
wobei die Summe aus n und m 3 bis 30 ist.
Weitere bevorzugte Ausführungsformen sind wie vorstehend beschrieben.

Die vorliegende Erfindung betrifft weiterhin ein Alkoxylat, wobei das Alkoxylat quarternisiertes Derivat (AQ) 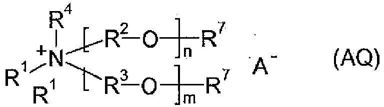 des Aminalkoxylats (A) ist, wobei
- R¹: ein verzweigter, aliphatischer Alkylrest C₉H₁₉ ist,
- R², R³, und R⁷: unabhängig voneinander Ethylen, Propylen, Butylen oder eine Mischung davon sind,
- R⁴: ein H, -OH, -OR⁶, -[R⁷-O]ₚ-R⁵ oder ein Sauerstoffanion ist,
- R⁵: ein H, C₁-C₆-Alkyl, C₂-C₆-Alkenyl, C₂-C₆-Alkinyl, -SO₃R^{a}, -P(O)OR^{b}OR^{c}, -CH₂CO₂R^{d}, oder -C(O)R^{e} ist,
- R⁶: ein C₁-C₆-Alkyl, C₂-C₆-Alkenyl, oder C₂-C₆-Alkinyl ist,
- R^{a} und R^{d}: unabhängig voneinander ein H, anorganische oder organische Kationen sind,
- R^{b} und R^{c}: unabhängig voneinander ein H, anorganische oder organische Kationen, C₁-C₆-Alkyl, C₂-C₆-Alkenyl oder C₂-C₆-Alkinyl sind,
- R^{e}: C₁-C₂₂-Alkyl, C₂-C₂₂-Alkenyl, C₂-C₂₂-Alkinyl, C₆-C₂₂-Aryl, C₇-C₂₂-Alkylaryl ist,
- n, m und p: unabhängig voneinander ein Wert von 1 bis 30 haben, und
- A-: ein landwirtschaftlich akzeptables Anion oder, falls R³ ein Sauerstoffanion ist, A⁻nicht vorhanden ist,
wobei die Summe aus n, m und p 3 bis 80 ist.

Dabei ist A⁻ bevorzugt ein Halogenid (wie Chlorid oder Bromid), Phosphat, Sulfat oder ein anionisches Pestizid. Besonders bevorzugt ist A⁻ ein anionisches Pestizid, wie Glyphosatanion oder Glufosinatanion. Weitere bevorzugte Ausführungsformen sind wie vorstehend beschrieben.

Die vorliegende Erfindung betrifft weiterhin Verfahren zur Herstellung des Aminalkoxylats (A) oder eines quartärnisierten Derivats (AQ) des Aminalkoxylats (A) umfassend die Alkoxylierung von Aminen R¹-NH₂ mit Ethylenoxid, Propylenoxid, Butylenoxid oder eine Mischung davon.

Das Amin R¹-NH₂ ist ein wichtiges Zwischenprodukt zur Herstellung des Aminalkoxylats (A) oder eines quartärnisierten Derivats (AQ) des Aminalkoxylats (A). Bevorzugte Ausführungsformen von R¹ sind wie oben beschrieben. Die Amine R¹-NH₂ können durch Umsetzung von Ammoniak mit Alkoholen R¹-OH hergestellt werden. Geeignete Katalysatoren und Reaktionsbedingungen sind in US 5,808,158 beschrieben.

Ein weiterer Gegenstand der vorliegenden Erfindung ist das Amin R¹-NH₂, wobei R¹ ein Gemisch verschiedener verzweigter, aliphatischer Alkylreste C₉H₁₉ ist, wobei der mittlere Verzweigungsgrad von R¹ 1,05 bis 1,8 beträgt, und wobei der Verzweigungsgrad als die Zahl der Methylguppen in einem Molekül abzüglich 1 definiert ist.

Die Alkohole R¹-OH sind allgemein bekannt (z.B. CAS Nr. 27458-94-2) und kommerziell erhältlich, beispielsweise als Isononanol INA von Evonik Oxeno GmbH, Isononanol von OXEA Corporation, oder Nonanol N von BASF SE. Der Gehalt an Isononanol liegt meist bei mindestens 70 Gew.%, bevorzugt mindestens 95 Gew.% und insbesondere bei mindestens 99 Gew.%.

Die Alkohole R¹-OH können grundsätzlich nach jedem beliebigen Verfahren synthetisiert werden, sofern sie jeweils den beschriebenen Verzweigungsgrad aufweisen.

Bevorzugt kann R¹-OH hergestellt werden ausgehend von einem Butene enthaltenden Kohlenwasserstoffgemisch, beispielsweise wie in WO 2000/63151 beschrieben:
a) In einem ersten Schritt werden Butene zu einem Gemisch isomerer Octene dimerisiert.
b) Das Octengemisch aus a) wird anschließend zu C9-Aldehyden hydroformyliert.
c) Der Aldehyd aus b) wird zu Isononanolen.

Die Einzelheiten dieser Synthese sind dem Fachmann allgemein bekannt, beispielsweise aus WO 2000/63151 [Schritt a) siehe Seite 6, Zeile 2 bis Seite 8, Zeile 7; Schritt b) siehe Seite 8, Zeile 9 bis Seite 9, Zeile 30; Schritt c) siehe Seite 9, Zeile 32 bis Seite10, Zeile 16). Ein besonders geeigneter Alkohol R¹-OH kann wie in WO 2000/63151 Beispiel 1, Verfahrensschritt 1 und 2 (Seite 13, Zeile 35 bis Seite 14, Zeile 33) beschrieben hergestellt werden.

Die Alkoxylierung kann durch starke Basen, wie Alkalihydroxide und Erdalkalihydroxide, Brönstedsäuren oder Lewissäuren, wie AlCl₃, BF₃. katalysiert werden. Für eng verteilte Alkoholalkoxylate können Katalysatoren wie Hydrotalcit oder DMC verwendet werden. Die Alkoxylierung erfolgt vorzugsweise bei Temperaturen im Bereich von etwa 80 bis 250 °C, bevorzugt etwa 100 bis 220 °C. Der Druck liegt vorzugsweise zwischen Umgebungsdruck und 600 bar. Gewünschtenfalls kann das Alkylenoxid eine Inertgasbeimischung, z. B. von etwa 5 bis 60 %, enthalten.

Das quarternisierte Derivats (AQ) des Aminalkoxylats (A) kann in einem weiteren Reaktionsschritt aus dem Aminalkoxylat (A) durch Quartärnisierung hergestellt werden. Zur Einführung des eines Sauerstoffanions in das Aminalkoxylat (A) kann es oxidiert werden, beispielsweise durch Umsetzung der Aminogruppe mit Wasserstoffperoxid, Persäuren (wie meta-Chlorperbenzoesäure oder Peressigsäure) oder Peroxomonoschwefelsäure.

Die Herstellung der quarternisierten Derivate (AQ) mit R⁴ = H kann durch einfache Protonierung von Ausgangsverbindungen der Struktur (A) erfolgen. Die Herstellung der quarternisierten Derivate (AQ) mit R⁴ = OH kann durch einfache Protonierung von Ausgangsverbindungen (AQ) mit R⁴ = Sauerstoffanion erfolgen. Zur Protonierung sind als Säuren organische Säuren (z.B. C₁ bis C₂₀ Carbonsäuren, insbesondere Benzoesäure) oder anorganische Säure (z.B. Salzsäure, Phosphorsäure, oder Schwefelsäure) geeignet. Ebenso geeignet sind auch H-azide Pestizide wie z.B. Glyphosate-säure oder Glyphosate-monosalze. Die Protonierung kann in einem separaten Syntheseschritt durchgeführt werden, so dass das quarterniesierte Derivat (AQ) isoliert werden kann. Es ist auch möglich die Protonierung durch Mischen der Ausgangsverbindungen mit einer oder mehreren Säuren in der Zusammensetzung oder in die Spritzbrühe durchzuführen.

Die vorliegende Erfindung betrifft auch die Verwendung des Aminalkoxylats (A) oder eines quarternisierten Derivats (AQ) des Aminalkoxylats (A) wie oben beschrieben als Hilfsmittel in Pestizid-haltigen Spritzbrühen. Bevorzugt ist das Hilfsmittel ein wirkungssteigerndes Hilfsmittel. Solche Wirkungssteigernden Hilfsmittel werden auch Adjuvantien genannt. Sie steigem oder beschleunigen die Wirkung von Pestiziden im Vergleich zur Wirkung des Pestizides in Abwesenheit des Adjuvants.

Die Vorteile der Erfindung sind eine hohe Stabilität der Formulierung und der Spritzbrühe, ein geringer Winddrift bei Sprühapplikation, eine hohe Haftung der Formulierung auf der Oberfläche der behandelten Pflanzen, eine Erhöhung der Löslichkeit der Pestizide in der Formulierung, eine erhöhte Permeation der Pestizide in die Pflanze und damit verbunden eine schnellere und erhöhte Wirksamkeit. Ein wichtiger Vorteil ist die geringe Toxizität der neuen Alkoxlyate, insbesondere die geringe Aquatoxizität. Ein weiterer Vorteil liegt in der einfachen Handhabung dieser Alkoxide, da z.B. in der Einarbeitung in Formulierungen keine Gelbildung auftritt.. Ein Vorteil ist auch die geringe Schadwirkung gegen Kulturpflanzen, d.h. eine geringe phytotoxische Effekte. Weitere Vorteil ist, dass verzweigte Nonanole großtechnisch verfügbar sind (Weltjahresproduktion mehrere Hunderttausend (!) Tonnen), da sie auch Vorprodukte von Weichmachern sind und aus kostengünstigen C4-Rohstoffen hergestellt werden können.

Nachfolgende Beispiele erläutern die Erfindung ohne sie einzuschränken.

### Beispiele

### Beispiel 1: Herstellung von verzweigtem C₉-Amin aus C₉-Alkohol

Als C₉-Alkohol wurde Nonanol N eingesetzt, das kommerziell erhältlich ist von BASF SE (ein Gemisch gesättigter, isomerer primärer Nonylalkohole, CAS 248-471-3, Siedebereich 195-203 °C (DIN 51751), Brechungsindex 1,4353, Dynamische Viskosität 12,95 mPas bei 20 °C (DIN 51562), Dichte 0,8335 g/ml (DIN 53217)).

Der C₉-Alkohol (1450 g) und ein Alkohol-Aminierungskatalysator (beschrieben in EP 696 572 A1; 4,8 Gew.% bezüglich C₁₇-Alkohol) wurde im Autoklaven vorgelegt und es wurde mit Stickstoff und Wasserstoff gespült. Dann wurde 2500 g Ammoniak beaufschlagt und unter Rühren erwärmt. Nach einer Reaktionszeit von zehn Stunden bei 235 °C und einem Druck H₂ von 280 bar wurde das so erhaltene C₉-Amin filtriert und am Rotationsverdampfer von Wasser befreit (Wassergehalt unter 0,1 Gew.%).

### Beispiel 2: Alkoxylierung von verzweigtem C₉-Amin mit 5, 7 oder 10 EO-Einheiten

Zunächst wurden 715 g (5 mol) C₉-Amin aus Beispiel 1 mit 23,1 g Wasser versetzt. Dann wurde nach Spülen mit Stickstoff bei 100 °C 440 g (10 mol) Ethylenoxid (EO) massengesteuert über 12h zudosiert. Danach wurde 6h bei 100°C nachgerührt. Anschließend wurde bei 90°C unter Vakuum 2h entwässert. Man erhielt eine Ausbeute von 1164g (=101% d.T.) mit einer Aminzahl von 239 mg KOH/g (243mgKOH/g Theorie). Im zweiten Schritt wurden zu 300 g (1,3mol) von diesem Vorprodukt 2,3 g 50%ige KOH zugegeben und bei 90°C unter Vakuum 2h entwässert. Nach Spülen mit Stickstoff wurden bei 120°C 286 g (6,5 mol) Ethylenoxid über 6h massengesteuert zudosiert. Danach wurde 6h bei 120°C nachgerührt. Das Produkt wurde unter Vakuum entgast und mit wenigen Tropfen Essigsäure neutralisiert. Man erhielt eine Ausbeute von 592g (101% d.T.) einer farblosen, niedrigviskosen Flüssigkeit (im folgenden "iso-C₉-7 EO"; Aminzahl: 119mg KOH/g (124mg KOH/g Theorie).
Analog zur Herstellung von iso-C₉-7 EO wurden auch iso-C₉-5 EO (d.h. ethoxyliert mit insgesamt 5 EO Gruppen) und iso-C₉-10 EO (d.h. ethoxyliert mit insgesamt 10 EO Gruppen) hergestellt ausgehend von dem Amin aus Beispiel 1.

### Beispiel 3 - Glyphosat SL auf Modellpflanzen Winterweizen bzw. Sojabohne

Für die Gewächshaustests wurden die Winterweizen bzw. Sojabohne in lehmigen Sandboden mit einer Aussaattiefe von 1-2 cm ausgesät bzw. getopft. Sobald die Pflanzen eine Wuchshöhe von 10 bis 25 cm (d.h. ca. 10 bis 21 Tage nach der Aussaat) erreicht hatten, wurden die Spritzbrühen auf die Pflanzen in der Spritzkabine appliziert.

Eine konzentrierte Formulierung enthaltend Glyphosat Isoproylammonium gelöst in Wasser und Aminalkoxylat aus Beispiel 2 wurde mit VE-Wasser verdünnt und wurden mit einer Wasseraufwandmenge von 375 l/ha ausgebracht (140 g bzw. 280 g Glyphosat/ha und je 300 g Aminalkoxylat/ha). Als Vergleich wurde Genamine® T-150 ("C_{16/18}-Amin-15 EO", kommerziell erhältlich von Clariant, Deutschland) eingesetzt, das ein mit 15 Ethylenoxid alkoxyliertes aliphatische C_{16/18}-Amin ist. Die Temperaturen in der 3 bis 4 Wochen dauernden Versuchsperiode betrugen zw. 18 - 35 ° C. Während dieser Zeit wurden die Versuchspflanzen optimal bewässert, wobei die Nährstoffversorgung über das Gießwasser erfolgte.

Die Bewertung der herbiziden Aktivität erfolgte mittels Bonitur der behandelten Pflanzen im Vergleich zu den unbehandelten Kontrollpflanzen (Tabelle 1-3). Die Bewertungsskala reicht von 0 % bis 100 % Wirkung. 100 % Wirkung bedeutet vollständiges Absterben zumindest der oberirdischen Pflanzenteile. Demgegenüber bedeutet 0 % Wirkung, das keine Unterschiede zwischen behandelten und unbehandelten Pflanzen auftraten. Die Ergebnisse in Tabelle 1 und 2 belegen die erhöhte Wirksamkeit des Wirkstoffs durch Zusatz des Aminalkoxylats.

**Tabelle 1: Winterweizen Sorte Cubus**

| Aminalkoxylat | Glyphosat [g/ha] | Wirksamkeit [%] nach 14 Tagen | Wirksamkeit [%] nach 21 Tagen | Wirksamkeit [%] nach 28 Tagen |
|---|---|---|---|---|
| -^{a)} | 280 | 71 | 81 | 81 |
| -^{a)} | 140 | 64 | 73 | 73 |
| C_{16/18}-15 EO^{a)} | 280 | 86 | 100 | 100 |
| C_{16/18}-15 EO^{a)} | 140 | 68 | 78 | 78 |
| iso-C₉-5 EO | 280 | 90 | 100 | 100 |
| iso-C₉-5 EO | 140 | 80 | 98 | 98 |
| iso-C₉-7 EO | 280 | 84 | 99 | 100 |
| iso-C₉-7 EO | 140 | 75 | 92 | 90 |
| iso-C₉-10 EO | 280 | 88 | 100 | 100 |
| iso-C₉-10 EO | 140 | 80 | 94 | 94 |

| | | | | |
|---|---|---|---|---|
| a) Vergleichsversuch, nicht erfindungsgemäß. | | | | |

**Tabelle 2: Sojabohne Sorte Oxford**

| Aminalkoxylat | Glyphosat [g/ha] | Wirksamkeit [%] nach 14 Tagen | Wirksamkeit [%] nach 21 Tagen | Wirksamkeit [%] nach 28 Tagen |
|---|---|---|---|---|
| -^{a)} | 140 | 29 | 38 | 38 |
| C_{16/18}-15 EO^{a)} | 140 | 44 | 50 | 50 |
| iso-C₉-5 EO | 140 | 54 | 71 | 71 |
| iso-C₉-7 EO | 140 | 44 | 54 | 54 |
| iso-C₉-10 EO | 140 | 43 | 55 | 56 |

| | | | | |
|---|---|---|---|---|
| a) Vergleichsversuch, nicht erfindungsgemäß. | | | | |

## Patentansprüche

1. Zusammensetzung umfassend ein Pestizid mit mindestens einer H-aciden Gruppe oder dessen anionisches Salz und ein Alkoxylat, **dadurch gekennzeichnet, dass** das Alkoxlyat ein Aminalkoxylat (A) 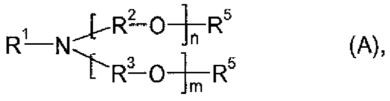 oder ein quaternisiertes Derivat (AQ) 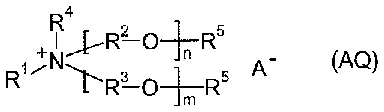 des Aminalkoxylats (A) ist, wobei
R¹ ein verzweigter, aliphatischer Alkylrest C₉H₁₉ ist,
R², R³, und R⁷ unabhängig voneinander Ethylen, Propylen, Butylen oder eine Mischung davon sind,
R⁴ ein H, -OH, -OR⁶, -[R⁷-O]ₚ-R⁵ oder ein Sauerstoffanion ist,
R⁵ ein H, C₁-C₆-Alkyl, C₂-C₆-Alkenyl, C₂-C₆-Alkinyl, -SO₃R^{a}, -P(O)OR^{b}OR^{c}, -CH₂CO₂R^{d}, oder -C(O)Rₑ ist,
R⁶ ein C₁-C₆-Alkyl, C₂-C₆-Alkenyl, oder C₂-C₆-Alkinyl ist,
R^{a} und R^{d} unabhängig voneinander ein H, anorganische oder organische Kationen sind,
R^{b} und R^{c} unabhängig voneinander ein H, anorganische oder organische Kationen, C₁-C₆-Alkyl, C₂-C₆-Alkenyl oder C₂-C₆-Alkinyl sind,
R^{e} C₁-C₂₂-Alkyl, C₂-C₂₂-Alkenyl, C₂-C₂₂-Alkinyl, C₆-C₂₂-Aryl, C₇-C₂₂-Alkylaryl ist,
n, m und p unabhängig voneinander ein Wert von 1 bis 30 haben, und
A⁻ ein landwirtschaftlich akzeptables Anion oder, falls R³ ein Sauerstoffanion ist, A⁻ nicht vorhanden ist.

2. Zusammensetzung nach Anspruch 1, wobei R², R³ und R⁷ unabhängig voneinander Ethylen, Ethylen und Propylen, Ethylen und Butylen, oder Ethylen, Propylen und Butylen sind.

3. Zusammensetzung nach Anspruch 1 oder 2, wobei A⁻ ein Halogenid, Phosphat, Sulfat oder anionisches Pestizid ist.

4. Zusammensetzung nach einem der Ansprüche 1 bis 3, wobei R⁴ ein H ist.

5. Zusammensetzung nach einem der Ansprüche 1 bis 4, wobei beim Aminalkoxlat (A) die Summe aus n und m 2 bis 40, und bei dessen quaternisiertem Derivat (AQ) die Summe aus n, m und p 3 bis 80 ist.

6. Zusammensetzung nach einem der Ansprüche 1 bis 5, wobei der mittlere Verzweigungsgrad von R¹ 1,01 bis 2,5 beträgt, und wobei der Verzweigungsgrad als die Zahl der Methylgruppen in einem Molekül abzüglich 1 definiert ist.

7. Zusammensetzung nach einem der Ansprüche 1 bis 6, wobei das Pestizid Glyphosat oder Glufosinat umfasst.

8. Zusammensetzung nach einem der Ansprüche 1 bis 7, wobei das Pestizid Glyphosat oder Glufosinat, und zusätzlich ein weiteres Pestizid umfasst.

9. Aminalkoxylat (A) gemäß einem der Ansprüche 1 bis 4, wobei die Summe aus n und m 3 bis 30 ist.

10. Quaternisiertes Derivat (AQ) des Aminalkoxylats (A) gemäß einem der Ansprüche 1 bis 4, wobei die Summe aus n, m und p 3 bis 80 ist.

11. Verfahren zur Herstellung des Aminalkoxylats (A) gemäß Anspruch 9 oder eines quaternisierten Derivats (AQ) des Aminalkoxylats (A) gemäß Anspruch 10 umfassend die Alkoxylierung von einem Amin R¹-NH₂ mit Ethylenoxid, Propylenoxid, Butylenoxid oder einer Mischung davon.

12. Amin R¹-NH₂, wobei R¹ ein Gemisch verschiedener verzweigter, aliphatischer Alkylreste C₉H₁₉ ist, wobei der mittlere Verzweigungsgrad von R¹ 1,05 bis 1,8 beträgt, und wobei der Verzweigungsgrad als die Zahl der Methylguppen in einem Molekül abzüglich 1 definiert ist.

13. Verfahren zur Bekämpfung von phytopathogenen Pilzen und/oder unerwünschtem Pflanzenwuchs und/oder unerwünschtem Insekten- oder Milbenbefall und/oder zur Regulation des Wachstums von Pflanzen, wobei man die Zusammensetzung gemäß einem der Ansprüche 1 bis 8 auf die jeweiligen Schädlinge, deren Lebensraum oder die vor dem jeweiligen Schädling zu schützenden Pflanzen, den Boden und/oder auf unerwünschte Pflanzen und/oder die Nutzpflanzen und/oder deren Lebensraum einwirken lässt.

14. Saatgut enthaltend die Zusammensetzung gemäß einem der Ansprüche 1 bis 8.

15. Verwendung des Aminalkoxylats (A) oder eines quaternisierten Derivats (AQ) des Aminalkoxylats (A) gemäß einem der Ansprüche 1 bis 6 als Hilfsmittel in Pestizid-haltigen Spritzbrühen, wobei das Pestizid ein Pestizid mit mindestens einer H-aciden Gruppe oder dessen anionisches Salz ist.

## Claims

1. A composition comprising a pesticide having at least one H-acidic group or anionic salt thereof and an alkoxylate, wherein the alkoxylate is an amine alkoxylate (A) 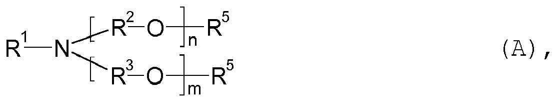 or a quaternized derivative (AQ) 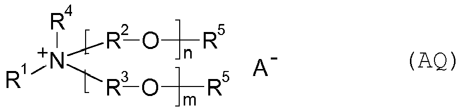 of the amine alkoxylate (A), where
R¹ is a branched aliphatic alkyl radical C₉H₁₉,
R², R³, and R⁷ independently of one another are ethylene, propylene, butylene or a mixture of these,
R⁴ is an H, -OH, -OR⁶, -[R⁷-O]ₚ-R⁵ or an oxygen anion,
R⁵ is an H, C₁-C₆-alkyl, C₂-C₆-alkenyl, C₂-C₆-alkynyl, -SO₃R^{a}, -P(O)OR^{b}OR^{c}, -CH₂CO₂R^{d} or -C(O)R^{e},
R⁶ is a C₁-C₆-alkyl, C₂-C₆-alkenyl or C₂-C₆-alkinyl,
R^{a} and R^{d} independently of one another are an H, inorganic or organic cations,
R^{b} and R^{c} independently of one another are an H, inorganic or organic cations, C₁-C₆-alkyl, C₂-C₆-alkenyl or C₂-C₆-alkynyl,
R^{e} is C₁-C₂₂-alkyl, C₂-C₂₂-alkenyl, C₂-C₂₂-alkinyl, C₆-C₂₂-aryl, C₇-C₂₂-alkylaryl,
n, m and p independently of one another have a value from 1 to 30, and
A⁻ is an agriculturally acceptable anion or, if R³ is an oxygen anion, A⁻ is absent.

2. The composition according to claim 1, wherein R², R³ and R⁷ independently of one another are ethylene, ethylene and propylene, ethylene and butylene, or ethylene, propylene and butylene.

3. The composition according to claim 1 or 2, wherein A⁻is a halide, phosphate, sulfate or anionic pesticide.

4. The composition according to any of claims 1 to 3, wherein R⁴ is an H.

5. The composition according to any of claims 1 to 4, wherein, in the amine alkoxylate (A), the total of n and m is from 2 to 40, and in its quaternized derivative (AQ) the total of n, m and p is from 3 to 80.

6. The composition according to any of claims 1 to 5, wherein the mean degree of branching of R¹ is from 1.01 to 2.5, and wherein the degree of branching is defined as the number of methyl groups in a molecule minus 1.

7. The composition according to any of claims 1 to 6, wherein the pesticide comprises glyphosate or glufosinate.

8. The composition according to any of claims 1 to 7, wherein the pesticide comprises glyphosate or glufosinate, and additionally a further pesticide.

9. An amine alkoxylate (A) according to any of claims 1 to 4, wherein the sum of n and m is from 3 to 30.

10. A quaternized derivative (AQ) of the amine alkoxylate (A) according to any of claims 1 to 4, wherein the sum of n, m and p is from 3 to 80.

11. A process for the preparation of the amine alkoxylate (A) according to claim 9 or a quaternized derivative (AQ) of the amine alkoxylate (A) according to claim 10, comprising the alkoxylation of an amine R¹-NH₂ with ethylene oxide, propylene oxide, butylene oxide or a mixture of these.

12. An amine R¹-NH₂, wherein R¹ is a mixture of different branched aliphatic alkyl radicals C₉H₁₉, wherein the mean degree of branching of R¹ is from 1.05 to 1.8, and wherein the degree of branching is defined as the number of methyl groups in a molecule minus 1.

13. A method of controlling phytopathogenic fungi and/or undesired vegetation and/or undesired insect or mite attack and/or for regulating the growth of plants, wherein the composition according to any of claims 1 to 8 is allowed to act on the respective pests, their environment or the plants to be protected from the respective pest, on the soil and/or on undesirable plants and/or the crop plants and/or their environment.

14. Seed, comprising the composition according to any of claims 1 to 8.

15. The use of the amine alkoxylate (A) or a quaternized derivative (AQ) of the amine alkoxylate (A) according to any of claims 1 to 6 as adjuvants in pesticide-comprising spray mixtures, wherein the pesticide is a pesticide having at least one H-acidic group or anionic salt thereof.

## Revendications

1. Composition comprenant un pesticide comportant au moins un groupe à H acide ou un sel anionique de celui-ci et un produit d'alcoxylation, **caractérisée en ce que** le produit d'alcoxylation est un produit d'alcoxylation d'amine (A) 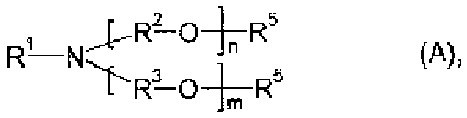 ou un dérivé quaternisé (AQ) 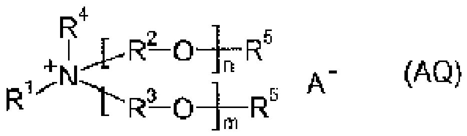 du produit d'alcoxylation d'amine (A), où
R¹ est un radical alkyle aliphatique en C₉-H₁₉ ramifié,
R², R³ et R⁷ représentent chacun indépendamment un groupe éthylène, propylène, butylène ou un mélange de ceux-ci,
R⁴ est un atome d'hydrogène, un groupe -OH, -OR⁶, -[R⁷-O]ₚ-R⁵ ou un anion oxygéné,
R⁵ est un atome d'hydrogène, un groupe alkyle en C₁-C₆, alcényle en C₂-C₆, alcynyle en C₂-C₆, -SO₃R^{a}, -P(O)OR^{b}OR^{c}, -CH₂CO₂R^{d} ou -C(O)R^{e},
R⁶ est un groupe alkyle en C₁-C₆, alcényle en C₂-C₆ ou alcynyle en C₂-C₆,
R^{a} et R^{d} représentent indépendamment l'un de l'autre un atome d'hydrogène, des cations organiques ou inorganiques,
R^{b} et R^{c} représentent indépendamment l'un de l'autre un atome d'hydrogène, des cations organiques ou inorganiques, un groupe alkyle en C₁-C₆, alcényle en C₂-C₆ ou alcynyle en C₂-C₆,
R^{e} est un groupe alkyle en C₁-C₂₂, alcényle en C₂-C₂₂, alcynyle en C₂-C₂₂, aryle en C₆-C₂₂, alkylaryle en C₇-C₂₂,
n, m et p ont chacun indépendamment une valeur de 1 à 30, et
A est un anion acceptable en agriculture, A n'étant pas présent lorsque R³ est un atome d'oxygène.

2. Composition selon la revendication 1, dans laquelle R², R³ et R⁷ représentent chacun indépendamment éthylène, éthylène et propylène, éthylène et butylène, ou éthylène, propylène et butylène.

3. Composition selon la revendication 1 ou 2, dans laquelle A⁻ est un halogénure, phosphate, sulfate ou pesticide anionique.

4. Composition selon l'une quelconque des revendications 1 à 3, dans laquelle R⁴ est un atome d'hydrogène.

5. Composition selon l'une quelconque des revendications 1 à 4, dans laquelle la somme de n et m dans le produit d'alcoxylation d'amine (A) vaut de 2 à 40, et la somme de n, m et p dans le dérivé quaternisé (AQ) de ce dernier vaut de 3 à 80.

6. Composition selon l'une quelconque des revendications 1 à 5, dans laquelle le degré moyen de ramification de R¹ vaut de 1,01 à 2,5, et le degré de ramification étant défini comme le nombre des groupes méthyle dans une molécule moins 1.

7. Composition selon l'une quelconque des revendications 1 à 6, dans laquelle le pesticide est le glyphosate ou le glufosinate.

8. Composition selon l'une quelconque des revendications 1 à 7, dans laquelle le pesticide est le glyphosate ou le glufosinate, et comprend en plus un autre pesticide.

9. Produit d'alcoxylation d'amine (A) selon l'une quelconque des revendications 1 à 4, dans lequel la somme de n et m vaut de 3 à 30.

10. Dérivé quaternisé (AQ) du produit d'alcoxylation d'amine (A) selon l'une quelconque des revendications 1 à 4, dans lequel la somme de n, m et p vaut de 3 à 80.

11. Procédé pour la préparation du produit d'alcoxylation d'amine (A) selon la revendication 9 ou d'un dérivé quaternisé (AQ) du produit d'alcoxylation d'amine (A) selon la revendication 10, comprenant l'alcoxylation d'une amine R¹-NH₂ avec de l'oxyde d'éthylène, de l'oxyde de propylène, de l'oxyde de butylène ou un mélange de ceux-ci.

12. Amine R¹-NH₂, dans laquelle R¹ est un mélange de divers radicaux alkyle aliphatiques en C₉-H₁₉ ramifiés, le degré moyen de ramification de R¹ valant de 1,05 à 1,8, et le degré de ramification étant défini comme le nombre des groupes méthyle dans une molécule moins 1.

13. Procédé pour la lutte contre des champignons phytopathogènes et/ou la croissance de plantes indésirables et/ou l'attaque par des insectes ou des acariens indésirables et/ou pour la régulation de la croissance de plantes, dans lequel on fait agir la composition selon l'une quelconque des revendications 1 à 8 sur les nuisibles respectifs, leur habitat ou sur les plantes à protéger contre le nuisible respectif, sur le sol et/ou sur les plantes indésirables et/ou sur les plantes utiles et/ou leur habitat.

14. Semence contenant la composition selon l'une quelconque des revendications 1 à 8.

15. Utilisation du produit d'alcoxylation d'amine (A) ou d'un dérivé quaternisé (AQ) du produit d'alcoxylation d'amine (A) selon l'une quelconque des revendications 1 à 6, en tant qu'adjuvant dans des bouillies à pulvériser contenant un pesticide, le pesticide étant un pesticide comportant au moins un groupe à H acide ou un sel anionique de celui-ci.
